(19) Europäisches Patentamt
European Patent Office
Office européen des brevets

(11) Veröffentlichungsnummer: **0 230 922**
**A2**

# (12) EUROPÄISCHE PATENTANMELDUNG

(21) Anmeldenummer: 87100501.3

(22) Anmeldetag: 16.01.87

(51) Int.Cl.³: **A 61 K 37/02**

(30) Priorität: 21.01.86 DE 3601562
01.03.86 DE 3606798

(43) Veröffentlichungstag der Anmeldung:
05.08.87 Patentblatt 87/32

(84) Benannte Vertragsstaaten:
AT BE CH DE ES FR GB GR IT LI LU NL SE

(71) Anmelder: BOEHRINGER INGELHEIM KG

D-6507 Ingelheim am Rhein(DE)

(84) Benannte Vertragsstaaten:
AT BE CH DE ES FR GB GR IT LI LU NL SE

(71) Anmelder: Boehringer Ingelheim International G.m.b.H

D-6507 Ingelheim am Rhein(DE)

(84) Benannte Vertragsstaaten:
GB

(72) Erfinder: Roos, Otto, Dr. Dipl.-Chem.
Elsheimer Strasse 36
D-6501 Schwabenheim(DE)

(72) Erfinder: Lösel, Walter, Dr. Dipl.-Chem.
Im Herzenacker 26
D-6535 Gau-Algesheim(DE)

(72) Erfinder: Schnorrenberg, Gerd, Dr. Dipl.-Chem.
Ernst-Ludwig-Strasse 66a
D-6535 Gau-Algesheim(DE)

(72) Erfinder: Arndts, Dietrich, Dr.
Mühlstrasse 7
D-6531 Appenheim(DE)

(72) Erfinder: Speck, Georg, Dr. Dipl.-Chem.
Rheinstrasse 13
D-6507 Ingelheim/Rhein(DE)

(72) Erfinder: Streller, Ilse, Dr.
Waldstrasse 16
D-6534 Stromberg(DE)

(72) Erfinder: Hinzen, Dieter, Prof. Dr.
Konrad-Adenauer-Strasse 26
D-6501 Zornheim(DE)

(72) Erfinder: Schingnitz, Günter, Dr.
Unter den Gärten 18
D-6550 Bad Kreuznach 14(DE)

(72) Erfinder: Lehr, Erich, Dr.
In der Toffel 5
D-6531 Waldalgesheim(DE)

**(54) Verwendung von Peptiden zur Herstellung von Mitteln zur Behandlung von Coronarerkrankungen oder organischem Hirnsyndrom.**

(57) Die Verwendung einer Verbindung gemäß Formel I

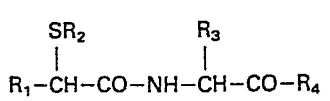

I

oder ihre Säureadditionssalze, zur Behandlung von Coronarerkrankungen oder organischem Hirnsyndrom.

EP 0 230 922 A2

Zahlreiche Angiotensin-Converting-Enzym-Hemmer (ACE-Hemmer) sind seit Jahren bekannt als Blutdrucksenker, deren Wirkung auf der Blockade des Renin-Angiotensin-Systems beruht. Besonders eingehend untersucht wurde das D-2-Methyl-3-mercaptopropanoyl-L-prolin, bekannt unter dem Generic Name CAPTOPRIL sowie der 1-N-[(S)-1-Carboxy-3-phenylpropyl]-S-alanyl-S-prolin-1'-ethylester (ENALAPRIL).

Seit einigen Jahren sind insbesondere für Captopril weitere Wirkungen aufgefunden worden. So erwies es sich als effektiv in der Behandlung der rheumatoiden Arthritis (1), des Raynaud-Syndroms (2), von Depressionen (3), der Herzinsuffizienz (4) und bei Schmerzzuständen, z.B. Migräne (5), (6).

Überraschenderweise wurde gefunden, daß die Verbindungen gemäß den europäischen Patentanmeldungen Nr. 85 110 941.3 und 85 112 356.2, die das Angiotensin Converting Enzym hemmen und eine starke und langanhaltende Blutdrucksenkung bewirken, insbesondere solche, die sich vom Dipeptid ableiten und deren Struktur sich grundlegend von der des Captoprils und des Enalaprils unterscheiden, eine sehr starke Cardioprotektion und Hirnprotektion entfalten.

Darüberhinaus zeigen sie eine Angiotensin II antagonistische Wirkung.
Gegenstand der vorliegenden Erfindung ist daher die Verwendung einer oder mehrerer der Verbindungen, die in der europäischen Patentanmeldung Nr. 85 110 941.3 und 85 112 356.2 beschrieben sind, d.h. Verbindungen der Struktur

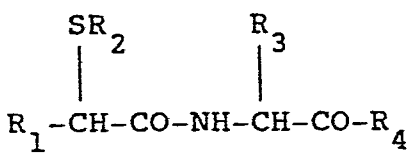

I

und ihre Salze für die Herstellung von pharmazeutischen Zusammensetzungen mit cardioprotektiver und hirnprotektiver Wirkung.

In der allgemeinen Formel I bedeutet

$R_1$ H, Phenyl, Benzyl, Phenethyl;

$R_2$ H, Acetyl, Benzoyl, Pivaloyl, Pivaloyloxymethyl,
3-Sulfonamido-4-chlor-benzoyl,
3-Sulfonamido-4-chlor-6-hydroxy-benzoyl,
3-Sulfonamido-4-chlor-5-[(furyl)-amino]-benzoyl,
2,3-Dichloro-4-(ß-phenyl-acryloyl)-phenoxy-acetyl,
$(C_1-C_2)$-Alkylaminocarbonyl,
$(C_1-C_2)$-Alkylaminothiocarbonyl oder den Rest der Formel

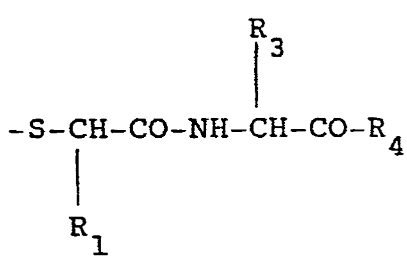

II

$R_3$ $-CH_3$ oder $-(CH_2)_p-NHR$, worin
p eine der Zahlen 1 - 4 und
R H, Acetyl, Benzoyl, Pivaloyl oder tert. butoxycarbonyl bedeuten,

$R_4$ den Rest einer der α-Aminosäuren

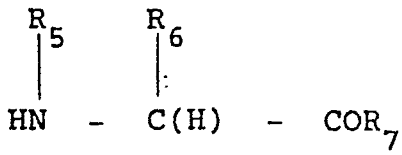

oder 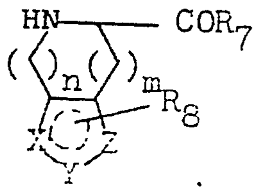

worin

$R_5$ H, $(C_1-C_4)$Alkyl, gerade oder verzweigt $(C_5-C_7)$-Cycloalkyl, wobei ein Phenylring ankondensiert sein kann, Phenyl, Phenyl-$(C_1-C_4)$alkyl, einen Heterocyclus-$(C_1-C_4)$alkylrest, wobei der Heterocyclus aus einem 5- oder 6-Ring mit 1 bis 2 der Heteroatomen O,S oder N besteht;

$R_6$ H, $(C_1-C_4)$Alkyl, gerade oder verzweigt, Phenyl, Phenyl-$(C_1-C_4)$alkyl oder einen Heterocyclus-$(C_1-C_4)$alkylrest, wobei der Heterocyclus aus einem 5- oder 6-Ring mit 1 bis 2 der Heteroatome O,S oder N besteht, oder

$R_5$ und $R_6$ zusammen mit dem N- und dem C-Atom einen 5-, 6- oder 7-gliedrigen Ring bilden, der gesättigt sein kann oder eine Doppelbindung enthalten kann, oder einen 4-, 5- oder 6-gliedrigen Ring, der ein oder zwei weitere der Heteroatome O,S oder N enthält;

$R_7$ OH, $(C_1-C_4)$-ω-Hydroxyalkyl, $(C_1-C_4)$-Alkoxy, Phenyl-$(C_1-C_4)$alkoxy, $(C_1-C_4)$alkylamino, Di-$(C_1-C_4)$alkylamino, eine der Gruppen

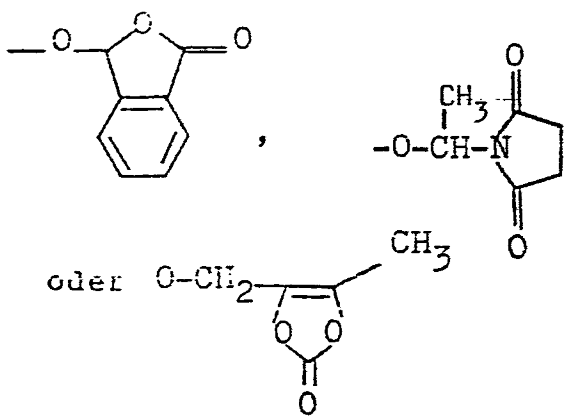

, , 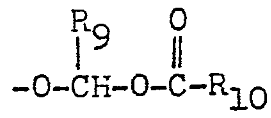 oder

oder eine α-Aminosäure, die peptidartig mit der =CO Gruppe des Moleküls verknüpft ist;
m und n jeweils 0,1 oder 2, wobei die Summe aus m und n 1 oder 2 ist, bedeuten,

$R_8$ H, $(C_1-C_4)$Alkyl, $(C_2-C_3)$ Alkenyl
$(C_2-C_3)$Alkinyl, gerade oder verzweigt,
Hydroxy, Nitro, Amino, $(C_1-C_4)$Alkoxy,
Mercapto, $(C_1-C_4)$Alkylthio,
Hydroxy-$(C_1-C_4)$alkyl,
Mercapto-$(C_1-C_4)$alkyl, F, Cl, Br,
Amino-$(C_1-C_4)$alkyl, Sulfonamido,
Methylendioxy, Fluor-$(C_1-C_4)$alkyl,
Chlor-$(C_1-C_4)$alkyl, Brom-$(C_1-C_4)$alkyl,
Cyano oder Trifluoromethyl;

$R_9$ H oder $CH_3$;
$R_{10}$ $(C_1-C_4)$Alkyl, gerade oder verzweigt, wobei der Alkylrest durch F, Cl, Br, $CF_3$, Phenyl oder Pyridyl substituiert sein kann;
unabhängig voneinander X, Y und Z = O,S, $=NR_{11}$, $=CR_{12}$, $=CHR_{12}$

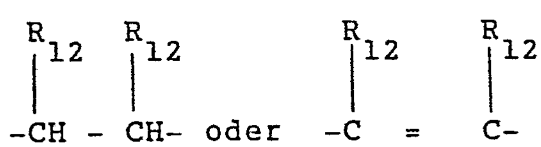

oder

$R_{11}$ H oder $(C_1-C_4)$Alkyl, gerade oder verzweigt,

$R_{12}$ H oder zusammen mit einem vicinal stehenden Rest $R_{12}$, einen Phenylring oder für m und n = 1, dessen Dihydroform mit der Doppelbindung in Konjugation zur C-terminalen Carboxygruppe, mit der Maßgabe,

a) daß nur einer der Reste X, Y und Z O,S

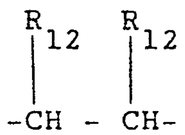

oder

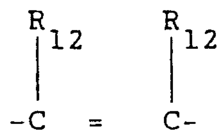

und einer oder zwei der Reste X, Y und Z $NR_{11}$ bedeuten können, und

b) wenn $R_3$ für die Gruppe $-(CH_2)_p-NHR$ steht, $R_1$ Benzyl und $R_2$ H, Acetyl, Benzoyl, Pivaloyl oder einen Rest der Formel II, bedeuten.

Die Verbindungen der Formel I weisen im allgemeinen mehrere Asymmetriezentren auf und liegen daher als Diastereomere oder in Form ihrer Racemate beziehungsweise ihrer racemischen Gemische vor. Die Erfindung umfaßt sowohl die Benutzung der racemischen Gemische als auch die der einzelnen Diastereomeren. Bevorzugt werden diejenigen Enantiomeren, bei denen die asymmetrischen C-Atome in der S-Konfiguration vorliegen. Die erwähnten Racemate können in üblicher Weise, z.B. durch fraktionierte Kristallisation, durch chemische oder durch biochemische Spaltung, in ihre sterisch einheitlichen Formen angereichert oder rein erhalten werden.

Die erfindungsgemäßen Säuren bilden Salze mit Säure als auch mit anorganischen oder organischen Basen. Beispiele für anorganische Basen sind Ammoniak, Alkalimetallhydroxyde wie Natrium- oder Kaliumhydroxyd oder Erdalkalimetallhydroxyde wie Calcium- oder Magnesiumhydroxyd; an organischen Basen seien genannt Dicyclohexylamin, N,N'-Dibenzyläthylendiamin, N,N'-Bis-(dehydroabietyl)-äthylendiamin, N-Methyl-D-glycamin, Arginin oder Lysin. Als Beispiel für zur Salzbildung geeigneter Säuren seien genannt, Halogensäure, Schwefelsäure oder Sulfonsäure.

Von den Verbindungen der allgemeinen Formel I können folgende als besonders interessant hervorgehoben werden:
- Verbindungen der Formel I, worin

$R_1$ H, Phenyl, Benzyl oder Phenethyl bedeutet;
$R_2$ H, Acetyl, Benzoyl, Pivaloyl, Pivaloyloxymethyl,
3-Sulfonamido-4-chlorbenzoyl,
$(C_1-C_2)$alkylaminocarbonyl
$(C_1-C_2)$alkylaminothiocarbonyl oder den Rest
der Formel

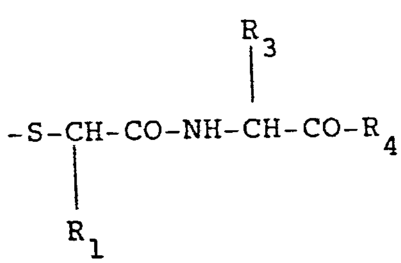

bedeutet;

0230922

$R_4$ den Rest einer der α-Aminosäuren

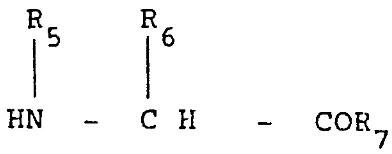

bedeutet,

worin,

$R_5$ $(C_5-C_7)$-Cycloalkyl

und

$R_6$ H bedeutet,

oder $R_5$ und $R_6$ zusammen mit dem N- und dem C-Atom einen 5-, 6-, oder 7-gliedrigen gesättigten Ring bilden, oder einen 4-, 5-, oder 6-gliedrigen Ring, der ein oder zwei weitere der Heteroatome O,S und N enthält;

$R_7$ OH, eine der Gruppen

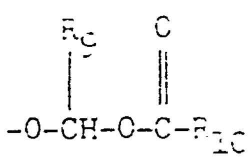

und 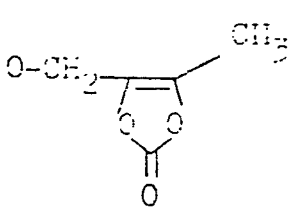

0230922

oder eine α-Aminosäure, die peptidartig mit der =CO Gruppe des Moleküls verknüpft ist, bedeutet; worin

$R_9$ H oder $CH_3$ ist;

$R_{10}$ $(C_1-C_4)$Alkyl, gerade oder verzweigt, wobei der Alkylrest durch F, Cl, Br, $CF_3$, Phenyl oder Pyridyl substituiert sein kann.

- Verbindungen der Formel I, worin
  $R_1$ Benzyl ist.

- Verbindungen der Formel I, worin
  $R_2$ Wasserstoff, Acetyl, Benzoyl oder Pivaloyloxymethyl, vorzugsweise Wasserstoff oder Pivaloyloxymethyl ist.

- Verbindungen der Formel I, worin
  $R_4$ der Rest

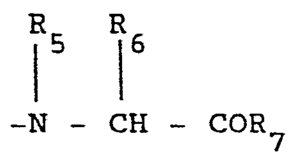

  ist, worin $R_5$ und $R_6$ zusammen mit dem N- und dem C-Atom einen 5-gliedrigen gesättigten Ring bilden oder einen 5-gliedrigen Ring, der als zweites Heteroatom S enthält.

- Verbindungen der Formel I, worin
  $R_7$ OH oder Pyvaloyloxymethoxy ist.

- Verbindungen der Formel I, worin
  $R_3$ $-CH_3$ ist.

0230922

- Verbindungen der Formel I, worin
  $R_3$ $-(CH_2)_4$ NHR ist, worin R wie in Anspruch 1 definiert ist, vorzugsweise H, Acetyl oder tert. Butoxycarbonyl bedeutet.

Spezifische Beispiele von Verbindungen der allgemeinen Formel I sind:

1. N-[N-(3-Phenyl-2-S-pivaloyloxymethylmercapto-propanoyl)-S-alanyl]-S-prolin-pivaloyloxymethylester;

2. N-[N-(3-Phenyl-2-S-benzoylmercaptopropanoyl)-S-alanyl]-S-prolin;

3. N-[N-(3-Phenyl-2-S-acetylmercaptopropanoyl)-S-alanyl]-S-prolin-pivaloyloxymethylester; und

4. N-[N-(3-Phenyl-2-S-mercaptopropanoyl)-S-alanyl]-S-prolin-pivaloyloxymethylester.

5. N,N'-[N,N'-{2,2'-S-Dithiobis-(3-phenylpropanoyl)}-bis-S-lysyl]-bis-S-prolinpivaloyloxymethylester

6. N,N-[N,N'-{2,2'-S-Dithiobis-(3-phenylpropanoyl)}-bis-S-lysyl]-bis-thiazolidin-4-S-carbonsäure

7. N,N-[N,N'-{2,2'-S-Dithiobis-(3-phenylpropanoyl)}-bis-S-lysyl]-bis-thiazolidin-4-S-carbonsäure-pivaloyl-oxy-methylester

8. N-[$N^{\alpha}$-(3-Phenyl-2-S-acetylmercaptopropanoyl)-S-lysyl]-S-prolin

9. N-[$N^{\alpha}$-(3-Phenyl-2-S-mercaptopropanoyl)-S-lysyl]-S-prolin

10. N-[N$^{\alpha}$-(3-Phenyl-2-R-mercaptopropanoyl)-S-lysyl]-S-prolin

11. N-[N$^{\alpha}$-(3-Phenyl-2-S-mercaptopropanoyl)-S-lysyl]-thiazolidin-4-S-carbonsäure

12. N-[N$^{\alpha}$-(3-Phenyl-2-S-acetylmercaptopropanoyl)-S-lysyl]-thiazolidin-4-S-carbonsäure

13. N-[N$^{\alpha}$-(3-Phenyl-2-S-acetylmercapto)-S-lysyl]-S-prolin-pivaloyloxymethylester

14. N-[N$^{\alpha}$-(3-Phenyl-2-S-acetylmercapto)-S-lysyl]-thiazolidin-4-S-carbonsäure-pivaloyloxymethylester

15. N,N'-[N,N'-{2,2'-S-Dithiobis-(3-phenylpropanoyl)}-bis-S-lysyl]-bis-S-prolin

16. N-[N-(2-Mercaptoacetyl)-alanyl]-prolin,

17. N-[N-(2-Phenyl-2-mercaptoacetyl)-alanyl]-prolin,

18. N-[N-(3-Phenyl-2-mercaptopropanoyl)-alanyl]-prolin,

19. N-[N-(4-Phenyl-2-mercaptobutyryl)-alanyl]-prolin,

20. N-[N-(2-Acetylmercaptoacetyl)-alanyl]-prolin,

21. N-[N-(2-Phenyl-2-acetylmercaptoacetyl)-alanyl]-prolin,

22. N-[N-(3-Phenyl-2-acetylmercaptopropanoyl)-alanyl]-prolin,

23. N,N'-[N,N'-{2,2'-dithiobis-(3-phenylpropanoyl)}-bis-S-alanyl]-bis-S-prolin,

24. N-[N-(3-Phenyl-2-S-{4-chlor-3-sulfon-amidobenzoyl}-mercaptopropanoyl)-S-alanyl]-S-prolin,

25. N-[N-(3-Phenyl-2-S-mercaptopropanoyl)-S-alanyl]-S-prolyl-S-phenylalanin,

CARDIOPROTEKTIVE WIRKUNG

Überraschenderweise wurde gefunden, daß die Verbindungen der allgemeinen Formel I und ihre Salze neben einer starken und langanhaltenden blutdrucksenkenden Wirkung, eine deutliche cardioprotektive Wirkung aufweisen, die wie folgt bestimmt wurde.

1. Bekanntlich ist der myocardiale $Ca^{2+}$ Gehalt ein Maß für die hypoxische bzw. die durch toxische Catecholamindosen hervorgerufene Herzschädigung (Higgins et al. Mol. Cell. Cardiol. 10. 427-438, 1984; Nakanishi et. al., Am. J. Physiol. 242, 437-449, 1982; Fleckenstein A., Vorträge der Erlanger Physiol. Tagung 1970, Edit. Keidel, Springer Verl. Berlin; Heidelberg, New York, 1971). Umgekehrt ist die Inhibition der hypoxischen oder Isoprenalin-bedingten myocardialen Calciumaufnahme ein Maß für die cardioprotektive Effektivität von Calciumantagonisten (Fleckenstein s.o.), von Calmodulininhibitoren (Higgins s.o.) und anderen Pharmaka, z.B. Betaadrenolytika (Arndts, Arzneimittelforschung, 25, 1279-1284 (1975)). Die cardioprotektive Wirkung wurde an wachen Ratten nach subkutaner oder peroraler Wirkstoffgabe anhand der von Arndts (s.o.) beschriebenen Methoden

festgestellt und die Wirkungsstärke der Testsubstanzen als $H_{50}$-Werte angegeben; dieser Wert entspricht der Dosis, die die durch eine Gabe von 30 mg/kg s.c. Isoprenalin bedingte myocardiale Radiocalciumaufnahme bis 50% hemmt. Hier erwiesen sich die vorliegenden Verbindungen als wirksamer als die bekannten Handelsprodukte Verapamil, Captopril, Enalapril und Diltiazem. Die Ergebnisse sind in Tabelle I dargestellt.

TABELLE 1

| Verbindung | $H_{50}$ mg/kg |
|---|---|
| 1 | 0,5 |
| 2 | 0,75 |
| 3 | 0,67 |
| Captopril | 1,52 |
| Enalapril | 1,59 |
| Verapamil | 1,0 |
| Diltiazem | 1,5 |

Verbindungen 1 - 3:

1. N-[N-(3-Phenyl-2-S-pivaloyloxymethylmercapto-propanoyl)-S-alanyl]-S-prolin-pivaloyloxymethylester;

2. N-[N-(3-Phenyl-2-S-benzoylmercaptopropanoyl)-S-alanyl]-S-prolin;

3. N-[N-(3-Phenyl-2-S-acetylmercaptopropanoyl)-S-alanyl]-S-prolin-pivaloyloxymethylester

Desweiteren zeigen die neuen Substanzen einen Schutz des Herzens gegen Schäden während Ischämie oder Hypoxie nach Konstriktion oder Verschluß eines oder mehrerer Coronargefäße.

2. In einer weiteren Testordnung wurde die Wirkung und der Mechanismus der Verbindungen auf die $Ca^{2+}$-Fluxen wie folgt nachgewiesen:
   Die Bestimmung des $Ca^{2+}$-Efflux wurde nach der Methode von C. van Breemen, P. Aaronson, R. Loutzenheiser und K. Meisheri (Chest 78, 157S-165S (1980) und von R. Casteels, S.9 Droogman (J. Physiol. 317, 263-279 (1981)) durchgeführt. Mit dem $Ca^{2+}$-Efflux wird die intrazelluläre $Ca^{2+}$-Freisetzung auf eine Rezeptorstimulation gemessen. Damit ist eine quantitative Beurteilung des Ausmaßes der Rezeptor-vermittelten Wirkung möglich.

   Untersuchungen zum $Ca^{2+}$-Efflux an der Kaninchen-Aorta ergaben, daß die Verbindungen der Formel I den durch Angiotensin II stimulierten $Ca^{2+}$-Efflux hemmen.

   Eine Beeinflussung des Angiotensin I-converting Enzymes spielt in dieser Testanwendung keine Rolle.

   Es handelt sich somit um einen direkten Effekt an Angiotensin II-Rezeptor.
   Damit ist erstmals eine direkte Hemmung des Angiotensin II-Rezeptors mit diesen Substanzen nachgewiesen.

Dies bedeutet, daß die blutdrucksenkende Wirkung dieser Verbindungen auch durch eine zusätzliche Angiotensin II-Rezeptorblockade interpretiert werden kann.

HIRNPROTEKTIVE WIRKUNG

Daneben weisen die neuen Substanzen eine deutliche ZNS-Wirksamkeit auf. Es ergaben sich Hinweise auf hirnprotektive Eigenschaften und auf eine günstige Beeinflussung mnestischer Funktionen, die wie folgt bestimmt wurden:

1. HYPOXIE-TOLERANZ, d.h. bei der Prüfung der Überlebensfähigkeit von Tieren in einer geschlossenen Kammer.

   Methodik

   Eine aus Plexiglas hergestellte, durchsichtige, mit einem Deckel verschließbare Kammer (28 cm x 40 cm x 20 cm; Gesamtvolumen 22,4 l) ist in 2 Hälften geteilt, in die je 10 männliche oder weibliche Mäuse (ChbI: NMRI; 20-25 g Körpergewicht) gesetzt werden. Die Kammer wird mit einem Gasgemisch, bestehend aus 96,5 % $N_2$ und 3,5 % $O_2$ durchströmt; das Strömungsvolumen beträgt 12 l/min.

   Eine der beiden Gruppen erhält Prüfsubstanzen, die andere das Vehikel (0,5 % Tylose), die 24 h, 16 h und 30 min vor dem Test oral appliziert werden. Etwa 6-7 min nach Beginn der Durchströmung der Kammer sterben die ersten Tiere. Das Experiment wird beendet, wenn in der Kontrollgruppe nur noch 2-3 Tiere Zeichen von Leben zeigen.
   Ohne die Tiere zu berühren, wird 15 min gewartet und dann die Zahl der überlebenden Tiere in den beiden Hälften der Kammern endgültig festgestellt. Die statistische Signifikanz der Differenz der überlebenden Tiere in beiden Gruppen kann mit dem "Exakten Test von Fisher für 2 x 2 Feldertafeln" festgestellt werden.

TABELLE 2

| Verbindung | Dosis (mg/kg)p.o. | Behandelte Tiere Überlebende % | Kontrolle unbehandelte Tiere überlebende % |
|---|---|---|---|
| 1. | 50 | 30 | 20 |
| | 50 | 80 | 30 |
| | 30 | 80 | 40 |
| | 10 | 100 | 20 |
| 2. | 50 | 90 | 20 |
| | 50 | 30 | 30 |
| | 30 | 60 | 30 |
| 3. | 50 | 100 | 20 |
| | 50 | 80 | 20 |
| | 30 | 30 | 20 |
| | 10 | 50 | 30 |
| | 10 | 50 | 20 |
| 4. | 50 | 70 | 20 |
| | 50 | 100 | 40 |
| | 30 | 30 | 10 |
| | 10 | 90 | 20 |

Verbindungen 1 - 4:

1. N-[N-(3-Phenyl-2-S-pivaloyloxymethylmercapto-propanoyl)-S-alanyl]-S-prolin-pivaloyloxymethylester;

2. N-[N-(3-Phenyl-2-S-benzoylmercaptopropanoyl)-S-alanyl]-S-prolin

3. N-[N-(3-Phenyl-2-S-acetylmercapto propanoyl)-S-alanyl]-S-prolin-pivaloyloxymethylester;

4. N-[N-(3-Phenyl-2-S-mercaptopropanoyl)-S-alanyl]-S-prolin-pivaloyloxymethylester

2. Scopolamin-Test

Methodik

Durch den cholinerg-muskarinen Antagonisten Scopolamin wird bei Mensch und Tier eine vorübergehende Störung des Übergangs von Inhalten des Kurzzeitgedächtnisses in das Langzeitgedächtnis hervorgerufen. Die auch klinisch bei gesunden Probanden durch Scopolamin auslösbaren, mnestischen Störungen sind in ihrer Ausprägung den psychopathologischen Veränderungen bei Patienten mit organischem Hirnsyndrom ähnlich (Drachman & Leavitt, 1974).

Der Scopolamin-Test wird an Gruppen von jeweils 10 männlichen, nicht nüchternen, naiven Mäusen (ChbI:NMRI; Körpergewicht 35 g) mit Hilfe einer passiven Vermeidereaktion durchgeführt. Das Testgerät besteht aus einer Skinner Box (30 cm x 26 cm x 29 cm) mit einem elektrifizierbaren Gitterboden und einer Plastikplattform (5 cm x 5 cm) an einer Seitenwand. Die Tiere werden einzeln in die Skinner Box gesetzt. Nach einer Orientierungsperiode von 30 sec werden kurze, unterbrochene Stromstöße von ca. 0,8 mA so oft an den Gitterboden der Box gelegt, bis das Tier gelernt hat, daß das Aufsuchen und Verweilen auf der Plastikplattform vor dem Stromstoß Sicherheit gewährt. Tiere, welche dies innerhalb von 120 sec nicht lernen, werden nicht weiter verwendet. Die anderen Mäuse werden unmittelbar nach Erlernen der passiven Vermeidereaktion in drei Gruppen (10 Tiere pro Gruppe) eingeteilt. Eine Gruppe erhält Scopolaminhydrobromid (0,6 mg/kg s.c.) und oral

Vehikel (0,5 % Tylose); die zweite Gruppe Scopolaminhydrobromid sowie die zu testende Substanz per os in 0,5 % Tylose und die dritte Gruppe lediglich 0,5 % Tylose p.o.. Eine Stunde nach Applikation wird jedes Tier einzeln auf die Plattform gesetzt, um das Erinnerungsvermögen an die erlernte Vermeidereaktion zu prüfen. Das Kriterium besteht in der Bestimmung, ob die Maus während mindestens 60 sec auf der Plattform verbleibt (Ja-Antwort) oder nicht (Nein-Antwort).

Etwa 75 % der Tiere, welche ausschließlich mit 0,5 % Tylose behandelt wurden, verbleiben bei der abschließenden Prüfung länger als 60 sec auf der Plattform: ein Ergebnis, welches als Ausdruck des guten Erinnerungsvermögens an die erlernte Erfahrung gedeutet werden kann. Nach Gabe von Scopolamin und Tylose verbleiben durchschnittlich etwa 25 % der Tiere auf der Plattform; dies zeigt die erhebliche Beeinträchtigung des Erinnerungsvermögens nach Gabe von Scopolamin.

TABELLE 3

| Verbindung | Dosis | %der Tiere bei welcher die Scopolamin-Wirkung antagonisiert wurde |
|---|---|---|
| 1. | 30 | 40 |
| | 20 | 40 |
| | 10 | 40 |
| | 10 | 30 |
| | 5 | 80 |
| | 5 | 40 |
| 2. | 30 | 50 |
| | 30 | 20 |
| | 10 | 50 |
| | 10 | 30 |
| | 5 | 50 |
| | 1 | 30 |
| 3. | 30 | 50 |
| | 30 | 20 |
| | 10 | 40 |
| | 10 | 80 |
| | 5 | 40 |
| | 1 | 60 |
| | 0,5 | 40 |

Verbindungen 1 - 3:

1. N-[N-(3-Phenyl-2-S-pivaloyloxymethylmercapto-propanoyl)-S-alanyl]-S-prolin-pivaloyloxymethylester;

2. N-[N-(3-Phenyl-2-S-benzoylmercaptopropanoyl)-S-alanyl]-S-prolin;

3. N-[N-(3-Phenyl-2-S-acetylmercapto propanoyl)-S-alanyl]-S-prolin-pivaloyloxymethylester:

3. Mäuse-Motilitätstest
Die vorliegenden Verbindungen zeigen bei niedriger Dosierung eine Steigerung der Motilität.

Ein Vergleichsversuch, zwischen N-[N-(3-Phenyl-2-S-acetylmercaptopropanoyl)-S-alanyl-]-S-prolin-pivaloyloxymethylester und dem Standard (Tylose), wurde mit je 6 Mäusen (Tierstamm CHbI:NMRI) durchgeführt.
Die Mäuse wurden im Lichtschrankenkäfig behalten, damit dieser Versuch als Nachtversuch gilt. Nach der Dosierung der Mäuse wurde jede Aktivität gezählt und die mittlere Aktivitätsrate pro Minute jeder Stunde bestimmt. Die resultierenden Ergebnisse sind in graphischer Form (Fig. 1) dargestellt.

Die vorliegenden Verbindungen können allein für die neuen Indikationen oder in Kombination mit anderen erfindungsgemäßen Wirkstoffen, gegebenenfalls auch in Kombination mit weiteren

pharmakologisch aktiven Wirkstoffen, z.B. mit antihypertensiv wirkenden Stoffen, zur Anwendung gelangen. Die Anwendung kann parenteral oder vorzugsweise oral erfolgen. Besonders geeignete Anwendungsformen sind beispielsweise Tabletten, Kapseln, Pulver usw.. Eine wirksame Dosis der Verbindungen liegt bei oraler Anwendung bei Menschen zwischen 5 und 100 mg, vorzugsweise zwischen 10 und 50 mg pro Tag.

Für die Anwendung in der Therapie werden die neuen Verbindungen mit üblichen pharmazeutischen Füll- oder Trägerstoffen, Streck-, Spreng-, Binde-, Gleit-, Dickungs- oder Verdünnungsmitteln gemischt.

Als pharmazeutische Zubereitungsformen kommen zum Beispiel Tabletten, Kapseln, Zäpfchen, Lösungen, Säfte, Emulsionen oder dispersible Pulver in Frage, wobei gewünschtenfalls weitere bekannte Wirkstoffe, z.B. Saluretica, Diuretica und/oder Antihypertonica zugefügt werden können.
Die vorliegenden Verbindungen können durch die in der deutschen Patentanmeldung Nr. P 34 32 307.4 und P 34 36 569.9 beschriebenen Verfahren hergestellt werden, d.h.

a) durch Kondensation einer Säure der allgemeinen Formel II

$$R_1 - \overset{SR_2}{\overset{|}{CH}} - COOH \qquad II$$

mit einem Dipeptid der allgemeinen Formel III

$$H_2N - \overset{R_3}{\overset{|}{CH}} - CO - R_4 \qquad III$$

b) Kondensation einer Verbindung der allgemeinen Formel IV

$$R_1 - \overset{SR_2}{\overset{|}{CH}} - CONH - \overset{R_3}{\overset{|}{CH}} - COOH \qquad IV$$

mit einer Aminosäure $R_4$.

c) Umsetzung einer Verbindung der allgemeinen Formel V

$$R_1 - \overset{Br}{\overset{|}{CH}} - CONH - \overset{R_3}{\overset{|}{CH}} - CO - R_4 \qquad V$$

mit einer Mercaptoverbindung der allgemeinen Formel VI

$$HS - R_2 \qquad VI$$

oder

d) Kondensation einer Verbindung der allgemeinen Formel VII

$$R_1 - \overset{Br}{\overset{|}{CH}} - CONH - \overset{R_3}{\overset{|}{CH}} - COOH \qquad VII$$

mit einer Aminosäure $R_4$ und anschließender Umsetzung des so erhaltenen Kondensationsproduktes der allgemeinen Formel V mit einer Mercaptoverbindung der allgemeinen Formel VI.

In den obigen Verfahren haben die Reste $R_1$, $R_2$, $R_3$ und $R_4$ die entsprechenden Bedeutungen wie unter Formel I definiert.

Die vorstehend beschriebenen Kondensationen erfolgen nach allgemein üblichen Methoden, wie sie in Houben-Weyl, Bd. XV/1,4, Auflage 1974 für die Synthese von Peptiden beschrieben worden sind.
Nachfolgend werden Beispiele für einige pharmazeutische Zusammensetzungen unter Verwendung einer oder mehrerer Verbindungen der allgemeinen Formel I angegeben. Falls nicht ausdrücklich anders bezeichnet, handelt es sich bei den Teilen um Gewichtsteile.

HERSTELLUNG VON WIRKSTOFFEN GEMÄß FORMEL I

Beispiel 1

N-[N-(3-Phenyl-2-S-mercaptopropanoyl)-S-alanyl]-S-prolin-pivaloyloxymethylester

1,75 g (5 mMol) N-[N-(3-Phenyl-2-S-mercaptopropanoyl)-S-alanyl]-S-prolin werden in 30 ml Aceton gelöst und unter Rühren und Stickstoffatomosphäre nacheinander 500 mg (5 mMol) $KHCO_3$, 140 mg (0,84 mMol) KJ und 0,81 ml (5 mMol) Chlormethylpivalat zugesetzt. Die Mischung wird 3 Stunden am Rückfluß gekocht und nach Abkühlen abgesaugt. Das Filtrat wird im Vakuum eingeengt, der Rückstand in Essigester aufgenommen und nacheinander gewaschen mit Wasser, gesättigter $NaHCo_3$-Lösung, Wasser und gesättigter NaCl-Lösung. Man trocknet über $MgSO_4$ und destilliert das Lösungsmittel im Vakuum ab. Der ölige Rückstand (1,5 g = 69 % der Theorie) wird über Kieselgel chromatographiert (Laufzeit: Essigester, n-Hexan (2:1)) und dabei 2 Fraktionen erhalten: 0,7 g (= 32 % der Theorie) entsprechen der Titelverbindung; Rf = 0,28.
0,3 g (13,7 % der Theorie) entsprechen dem S-Alkylierungsprodukt N-[N-(3-Phenyl-2-S-pivaloyloxymethylmercaptopropanoyl)-S-alanyl]-S-prolin-pivaloyloxy methylester.

Beispiel 2

N-[$N^{\alpha}$-(3-Phenyl-2-S-acetylmercaptopropanoyl)-S-lysyl]-S-prolin

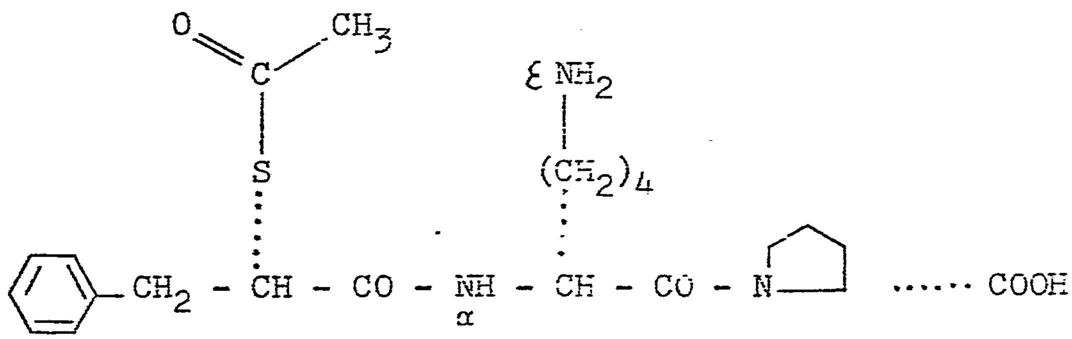

10,7 g (26,7 mMol) N-($N^{\varepsilon}$-tert.Butoxycarbonyl-S-lysyl)-S-prolin-tert.butylester und 6,11 g (26,7 mMol) 3-Phenyl-2-R-brompropionsäure werden in 270 ml wasserfreiem Dichlormethan gelöst und bei 0°C unter Rühren 5,5 g (26,7 mMol) N,N'-Dicyclohexylcarbodiimid zugegeben. Man rührt über Nacht bei Raumtemperatur weiter, saugt vom ausgefallenen Harnstoffderivat ab und engt das Filtrat im Vakuum ein. Der Rückstand wird in Essigester gelöst und vom Ungelösten abfiltriert. Die organische Phase wird nacheinander mit 5%iger $KHSO_4$-Lösung, $NaHCO_3$-Lösung und gesättigter NaCl-Lösung gewaschen und über $MgSO_4$ getrocknet. Nach Abdestillieren des Essigesters im Vakuum bleibt ein öliger Rückstand von 14 g (= 85,8 % der Theorie), dem NMR-spektroskopisch die Struktur des N-[$N^{\alpha}$-(3-Phenyl-2-R-brompropanoyl)-$N^{\varepsilon}$-tert.butoxycarbonyl-S-lysyl]-S-prolin-tert.butylesters zukommt.

Für den Acetylmercapto-Austausch werden 3,48 g (45,8 mMol) Thiolessigsäure in 120 ml wasserfreiem Ether gelöst und 4,62 g (45,8 mMol) Triethylamin, dann 14 g (22,9 mMol) der aus dem obigen Ansatz erhaltenen Bromverbindung bei 0°C zugetropft. Man kocht noch 90 Minuten am Rückfluß und läßt über Nacht bei Raumtemperatur stehen. Vom aus gefallenen Triethylaminhydrobromid wird abgesaugt, das Filtrat im Vakuum abdestilliert und dabei 15,5 g eines braunen Öls als Rückstand erhalten.

Die chromatographische Reinigung über Kieselgel (Laufmittel: Essigester, n-Hexan = 1:1) ergab 9,4 g (= 67,7 % d. Th.) des N-[$N^{\alpha}$-(3-Phenyl-2-S-acetyl-mercaptopropanoyl)-$N^{\varepsilon}$tert. butoxycarbonyl-S-lysyl] -S-prolin-tert.butylesters.

Zur Verseifung des tert. Butylesters und gleichzeitiger Abspaltung der $N^{\varepsilon}$-tert.Butoxycarbonyl-Schutzgruppe werden 5,5 g (9,08 mMol) des aus vorstehendem Ansatz und bei Raumtemperatur über Nacht gerührt. Nach Abdestillieren der trifluoressigsäure i.V. wird noch mehrmals in Dichlormethan gelöst und eingedampft. Der Rückstand wird in $NaHCO_3$-Lösung gelöst und mit Essigester ausgeschüttelt; die wäßrige Phase wird mit konzentrierter Salzsäure angesäuert und zweimal mit Dichlormethan extrahiert. Die vereinigten Dichlormethanextrakte werden mit Wasser und gesättigter NaCL-Lösung gewaschen, getrocknet und im Vakuum abdestilliert. Rückstand 0,5 g. Die wäßrige Phase wird mit NaCl gesättigt und erneut mit Dichlormethan extrahiert. Nach Trocknen über $MgSO_4$ und Eindampfen werden weitere 3 g, zusammen also 3,5 g, als erstarrter Schaum erhalten, der NMR-spektroskopisch der Struktur der Titelverbindung entspricht.
Rf = 0,22 (Butanol, Essigsäure, Wasser = 3:1:1, Silicalgel)

Beispiel 3

N-[$N^{\alpha}$-(3-Phenyl-2-S-mercaptopropanoyl)-N-acetyl-S-lysyl]-S-prolin

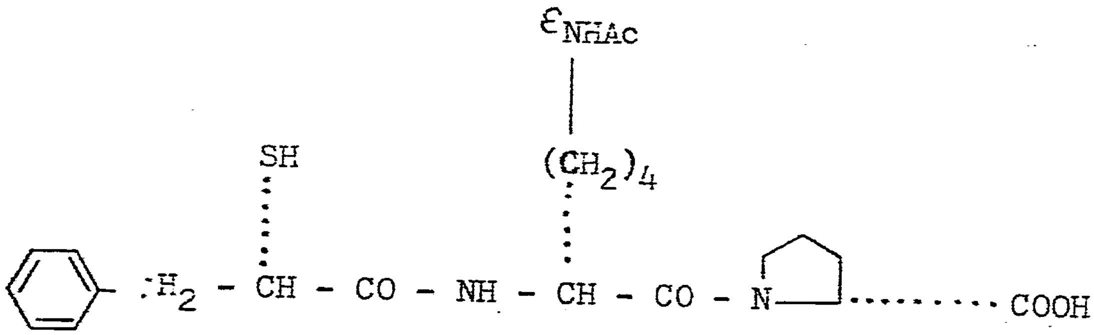

4,2 g (9,3 mMol)
N-[$N^{\alpha}$-(3-Phenyl-2-S-acetylmercaptopropanoyl)-S-lysyl]-S-prolin werden in 95 ml Wasser gelöst, mit 8,2 ml 5 n Natronlauge versetzt und 1,5 Stunden bei Raumtemperatur und $N_2$-Atmosphäre gerührt. Danach werden weitere 4,1 ml 5 n Natronlauge zugegeben und 1,5 Stunden weitergerührt. Die alkalische Lösung wird mit Essigester ausgeschüttelt, mit konzentrierter Salzsäure angesäuert und anschließend mit Dichlormethan extrahiert. Die organische Phase wird mit gesättigter NaCl-Lösung gewaschen, getrocknet und im Vakuum abdestilliert. Es werden 2,1 g (= 55,2 % der Theorie) der Titelverbindung als weißer erstarrter Schaum erhalten.
Rf = 0,49 (Butanol, Essigsäure, Wasser = 3:1:1, (Kieselgel $60F_{254}$ Merck).

0230922

Beispiel 4

N-[$N^\alpha$-(3-Phenyl-2-S-mercaptopropanoyl)-S-lysyl]-S-prolin

Zu einer Lösung von 0,989 g (13 mMol) Thiolessigsäure in 45 ml wasserfreiem Ether und 1,31 g (13 mMol) Triethylamin wird tropfenweise unter Stickstoff und Rühren eine Lösung von 3,7 g (6,5 mMol) N-[$N^\alpha$-3-phenyl-2-R-brompropanoyl)-$N^\varepsilon$-Tert.butoxycarbonyl)-S-lysyl]-S-prolin-methylester in 30 ml Essigester gegeben. Man kocht danach am Rückfluß, filtriert die vorher gekühlte Lösung und wäscht nacheinander mit verdünnter $KHSO_4$-Lösung, $NaHCO_3$-Lösung, Wasser und gesättigter NaCl-Lösung. Nach dem Trocknen über $MgSO_4$ wird das Lösungsmittel im Vakuum abdestilliert und als Rückstand 4,0 g eines gelblichen Öles erhalten. Die chromatographische Reinigung erfolgt über 120 g Kieselgel mit Essigester, n-Hexan = 2:1 als Elutionsmittel; es werden so 3,0 g (81,9 % der Theorie) reiner N-[$N^\alpha$-(3-Phenyl-2-S-acetyl-mercaptopropanoyl)-$N^\varepsilon$-tert.butoxycarbonyl-S-lysyl]-S-prolinmethylester als farbloses Öl erhalten. Rf = 0,26 (Essigester, n-Hexan = 2:1, Kieselgel $60_{254}$Merck).

Durch Schutzgruppenabspaltung (z.B. wie in Beispiel 30 beschrieben, wird 1,7 g (= 78,7 % der Theorie) des Titelproduktes erhalten.
Rf = 0,52 (Essigester, Butanol, Wasser, Essigsäure = 1:1:1:1, Kieselgel $60F_{254}$Merck).

Beispiel 5

N-[$N^{\alpha}$-(3-Phenyl-2-R-mercaptopropanoyl)-S-lysyl]-S-prolin

Das dem Beispiel 4 analoge RSS-Diastereomer wird erhalten, wenn man der R-Bromverbindung das entsprechende S-Isomer einsetzt, nämlich der N-[$N^{\alpha}$-(3-Phenyl-2-S-brompropanoyl)-$N^{\varepsilon}$-tert. butoxycarbonyl-S-lysyl]-S-prolin methylester.
Rf = 0,44 (n-Butanol, Eisessig, Wasser = 3:1:1 Kieselgel $60F_{254}$Merck).

Beispiel 6

N-[$N^{\alpha}$-(3-Phenyl-2-S-acetylmercaptopropanoyl)-S-lysyl]-S-prolin-(5-methyl-2-oxo-1,3-dioxolen-4-yl)-methylester

A. 2,6 g (4,7 mMol) N-[$N^{\alpha}$-(3-Phenyl-2-R-bromopropanoyl)-$N^{\varepsilon}$-tert. butoxycarbonyl-S-lysyl]-S-prolin werden in 400 ml Aceton gelöst und nacheinander 500,5 mg (5 mMol) $KHCO_3$, 141 mg (0,85 mMol) KJ und 965 mg (5 mMol) 4-Brommethyl-5-methyl-2-oxo-1,3-dioxolen zugegeben. Die Lösung wird in einer $N_2$-atmosphäre unter Rühren 3 Stunden am Rückfluß gekocht und nach abkühlen vom Ungelösten abfiltriert.

Nach Abdestillieren des Lösungsmittels wird der Rückstand in Essigester gelöst und mit Wasser, gesättigter $NaHCO_3$-Lösung, Wasser und gesättigter NaCl-Lösung gewaschen. Nach Trocknen über $MgSO_4$ wird der Essigester im Vakuum abdestilliert. Der Rückstand von 2,8 g wird über 90 g Kieselgel chromatographisch gereinigt (Elution mit Essigester, n-Hexan = 2:1). Es werden

2,3 g (= 73,4 % der Theorie) eines öligen Produktes erhalten, das NMR-spektroskopisch dem Prolin-(5-methyl-2-oxol-3-dioxolen-4-yl)-methylester derivat entspricht.
Rf = 0,68 (Essigester, n-Hexan = 2:1, Kieselgel $60F_{254}$ Merck).

B. Zum Austausch des Broms gegen die Acetylmercaptogruppe wird eine Lösung von 525 mg (6,9 mMol) Thiolessigsäure in 20 ml wasserfreiem Ether unter $N_2$-Atmosphäre und Eiskühlung langsam mit 0,7 g (6,9 mMol) Triäthylamin versetzt und anschließend tropfenweise unter Rühren zu einer Lösung von 2,3 g (3,5 mMol) der vorstehend genannten Bromverbindung in 20 ml wasserfreiem Ether und 10 ml Essigester gegeben. Man kocht danach noch 90 Minuten am Rückfluß, filtriert die vorher gekühlte Lösung und wäscht nacheinander mit verdünnter $KHSO_4$-Lösung, $NaHCO_3$-Lösung, Wasser und gesättigter NaCl-Lösung. Nach dem Trocknen über $MgSO_4$ wird das Lösungsmittel im Vakuum abdestilliert und als Rückstand 2,3 g eines gelblichen Öls erhalten. Die chromatographische Reinigung erfolgt über 7,5 g Kieselgel mit Essigester, n-Hexan = 2:1 als Elutionsmittel. Es werden so 1,7 g (= 74,4 % der Theorie) von $N-[N^{\alpha}$-(3-Phenyl-2-S-acetylmercaptopropanoyl)-$N^{\varepsilon}$-tert. butoxycarbonyl)-S-lysyl]-S-prolin-(5-methyl-2-oxo-1,3-dioxolen-4-yl)-methylester.

C. Zur Abspaltung der tert. butoxycarbonyl-$N^{\varepsilon}$-Schutzgruppe des Lysins werden 1,7 g (2,57 mMol) des Produktes aus Stufe B mit 40 ml Trifluoressigsäure 3 Stunden bei Raumtemperatur gerührt, anschließend im Vakuum zum Trocknen eingedampft und zur Entfernung restlicher Trifluoressigsäure nacheinander je dreimal in Aceton und in Chloroform gelöst und erneut

eingedampft. Es werden 2,3 g der Titelverbindung erhalten.

Beispiel 7

Die Titelverbindung aus den Beispielen 2 und 3 analog RSS-Diastereomeren nämlich
$N-[N^{\alpha}$-3-Phenyl-2-R-acetylmercaptopropanoyl)-S-lysyl]-S-prolin und
$N-[N^{\alpha}$-3-Phenyl-2-R-mercaptopropanoyl)-$N^{\varepsilon}$-acetyl-S-lysyl]-S-prolin werden erhalten, wenn man anstelle der R-Bromverbindung das entsprechende S-Isomere einsetzt, nämlich die 3-Phenyl-2-S-brompropionsäure.

Beispiel 8

N-[N-(3-Phenyl-2-S-mercaptopropanoyl)-S-alanyl]-S-prolin

Eine Lösung von 5,33 g (70 mMol) Thiolessigsäure in 150 ml wasserfreiem Ether wird unter Stickstoff und Eiskühlung langsam mit 7,1 g (70 mMol) Triethylamin versetzt und anschließend tropfenweise zu einer Lösung von 15,9 g (35 mMol) N-[N-(3-Phenyl-2-R-brompropanoyl)-S-alanyl]-S-prolin-tert. butylester in 75 ml wasserfreiem Ether unter Rühren gegeben. Man kocht danach noch 90 Minuten am Rückfluß, filtriert die vorher gekühlte Lösung und wäscht mit verdünnter $KHSO_4$- und $NaHCO_3$-Lösung, Wasser und gesättigter NaCl-Lösung. Nach dem Trocknen über $MgSO_4$ wird das Lösungsmittel abdestilliert und der ölige Rückstand (16,4 g) über Kieselgel chromatographiert (Laufmittel: Essigester, n-Hexan (2:1)). 13,1 g (= 83,4 % der Theorie) des Acetylmercapto-tert. butylesters werden als farbloses, zähes Öl erhalten; Rf = 0,41 (Essigester, n-Hexan = 2:1, Silicagel).

Zur Verseifung des tert.-Butylesters wird eine Lösung von 13,1 g (29,2 mMol) des obigen Esters in 65 ml Anisol und 130 ml Trifluoressigsäure 2 Stunden bei Raumtemperatur gerührt. Anschließend wird im Vakuum eingeengt und der Rückstand jeweils dreimal in ca. 100 ml Aceton, dann in Chloroform, gelöst und nachfolgend im Vakuum einrotiert. Der Rückstand wird in Dichlormethan gelöst und zweimal mit gesättigter $NaHCO_3$-Lösung extrahiert. Die vereinigten $NaHCO_3$-Lösungen werden mit Dichlormethan gewaschen, die wäßrige Phase mit konz. HCl angesäuert und mit Dichlormethan extrahiert. Die Dichlormethanlösung wird mit Wasser, gesättigter NaCl-Lösung gewaschen, über $MgSO_4$ getrocknet und im Vakuum eingedampft.

10,6 g (= 92,5 % der Theorie) der freien Säure, N-[N-(3-Phenyl-2-S-acetylmercaptopropanoyl)-S-alanyl]-S-prolin, werden als erstarrter Schaum isoliert; Rf = 0,6 (Chloroform, Methanol, Eisessig = 90:10:5, Silicagel).

Zur Verseifung des Thiolessigsäureesters werden zu 10,6 g (27 mMol) N-[N-(3-Phenyl-2-S-acetylmercaptopropanoyl)-S-alanyl]-S-prolin unter $N_2$-Atomosphäre und Rühren 250 ml $H_2O$ und 22 ml 5 n NaOH gegeben und die Lösung 1,5 Stunden bei Raumtemperatur belassen. Danach werden weitere 10 ml 5 n NaOH gegeben und bei der gleichen Temperatur 1,5 Stunden lang weitergerührt. Die wäßrige Lösung wird zweimal mit Dichlormethan gewaschen, mit halbkonzentrierter HCl angesäuert und dreimal mit Dichlormethan extrahiert. Die organische Phase wird mit gesättigter NaCl-Lösung gewaschen, über $MgSO_4$ getrocknet und eingedampft.
9,2 g (= 97,2 % der Theorie) der Titelverbindung werden als erstarrter Schaum isoliert, deren Struktur aufgrund der IR- und NMR-Daten bestätigt wurde.
Rf = 0,6 (Chloroform, Methanol, Eisessig = 90:10:5, Silicagel).

Die Ausgangsverbindungen werden wie folgt hergestellt:

3-Phenyl-2-R-brompropionsäure

Lit.: Chemistry of the Amino Acids, Vol. I, N.Y. (1961), p. 165; Ann 357, 1 (1907).

Abgewandelte Literaturvorschrift:
51,2 g (0,31 Mol) R-Phenylalanin werden nacheinander unter Rühren mit 620 ml 2,5 n $H_2SO_4$ und 125 g KBr (1,05 Mol) versetzt. Nach Abkühlen der Lösung auf 0°C werden portionsweise innerhalb von 80 Minuten32,75 g (00,475 Mol) $NaNO_2$ gegeben und 1 Stunde bei Raumtemperatur weitergerührt. Die entstandene Emulsion wird dreimal mit je ca. 200 ml Ether extrahiert, die organische Phase mit Wasser und gesättigter NaCl-Lösung gewaschen, über $MgSO_4$ getrocknet und der Ether abdestilliert. Der ölige Rückstand (63 g) wird über Kieselgel mit Essigester, n-Hexan (= 2:1) als Elutionsmittel chromatographisch gereinigt.
45,1 g der Titelverbindung werden als rötliches Öl erhalten, die Struktur und die optische Reinheit NMR-spektroskopisch bestätigt. Die Herstellung der S- und R,S-Analogen erfolgt nach der gleichen Vorschrift.

N-(3-Phenyl-2-R-brompropanoyl)-S-alanin

Zu einer Lösung von 22,9 g (0,1 Mol) 3-Phenyl-2-R-brompropionsäure, 13,9 g (0,1 Mol) S-Alaninmethylesterhydrochlorid und 10 g (0,1 Mol) Triethylamin in 500 ml wasserfreiem Dichlormethan werden unter Rühren bei 0°C 20,6 g (0,1 Mol) N,N'-Dicyclohexylcarbodiimid gegeben, 15 Minuten bei 0°C, dann über Nacht bei Raumtemperatur, weitergerührt. Nach Absaugen und Abdestillieren des Lösungsmittels wird der Rückstand in Essigester gelöst und nach Kühlung abfiltriert. Die Essigesterlösung wird

nacheinander mit verdünnter $KHSO_4$-, gesättigter $NaHCO_3$-Lösung, Wasser, gesättigter NaCl-Lösung gewaschen und über $MgSO_4$ getrocknet. Nach Abdestillieren des Essigesters wird der ölige Rückstand (29,9 g) über Kieselgel chromatographisch (n-Hexan, Essigester = 2:1) und dabei 23,9 g (= 76 % der Theorie; enspr. 76 mMol) der Titelverbindung als Methylester erhalten.

Zu einer Verseifung löst man in 150 ml Methanol, kühlt auf 0°C und setzt unter Rühren 80 ml 1 n NaOH (80 mMol) zu. Nach dreistündigem Rühren bei Raumtemperatur wird im Vakuum eingedampft, der Rückstand mit Wasser verdünnt und dreimal mit Dichlormethan extrahiert. Nach Abziehen des in der Wasserphase gelösten Dichlormethans im Vakuum wird mit konzentrierter HCl angesäuert und die ausgefallenen Kristalle abfiltriert. Ausbeute: 17,8 g (= 78 % der Theorie) der Titelverbindung vom Fp. 156-157°C werden erhalten.
Rf = 0,7 (Chloroform, Methanol, Eisessig 90:10:5, Silicagel).

Analog werden das S S- und (R,S) S-Diastereomere erhalten.
Ein NMR-spektroskopischer Vergleich der S S- und R,S-Diastereomeren zeigt den stereoselektiven Verlauf der Synthese.

N-[N-(3-Phenyl-2-R-brompropanoyl)-S-alanyl]-S-prolin-tert. butylester

a) 17,8 g (59,3 mMol) N-(3-Phenyl-2-R-brompropanoyl)-S-alanin und 10,1 g (59,3 mMol) S-Prolin-tert. butylester werden in 250 ml wasserfreiem Dichlormethan gelöst und unter Rühren und Kühlen (0°C) mit 12,2 g (59,3 mMol) N,N'-Dicyclohexylcarbodiimid versetzt. Nach Absaugen des Dicyclohexylcarbodiimids wird das Lösungsmittel im Vakuum abdestilliert, der ölige Rückstand in Essigester gelöst und einige Zeit kaltgestellt. Nach erneutem Absaugen wird nacheinander mit verdünnter $KHSO_4$-Lösung, gesättigter $NaHCO_3$-Lösung, Wasser und gesättigter NaCl-Lösung gewaschen und nach Trocknen über $MgSO_4$ das Lösungsmittel im Vakuum abdestilliert. Der ölige Rückstand (24,2 g) wird über Kieselgel chromatographiert (Laufmittel: Essigester, n-Hexan (2:1)).
15,9 g (= 59,1 % der Theorie) der Titelverbindung können als farbloses, zähes Öl isoliert werden, das NMR-spektroskopisch und chromatographisch einheitlich erscheint.
Rf = 0,45 (Essigester, Hexan (2:1), Silicagel).

Analog werden die S S S- und R,S S S-analogen Diastereomeren erhalten.

b) Eine Lösung von 2,29 g (10 mMol) 3-Phenyl-2-S-brompropionsäure, 2,78 g (10 mMol) S-Alanyl-S-prolin-tert. butylester-hydrochlorid und 1 g (10 mMol) Triethylamin werden in 100 ml wasserfreiem Dichlormethan gelöst und unter Rühren und Kühlen (0°C) mit 2,06 g (10 mMol) N,N'-Dicyclohexylcarbodiimid versetzt. Man rührt über Nacht bei Raumtemperatur weiter, kühlt und saugt vom ausgefallenen Dicyclohexylharnstoff ab. Das Lösungsmittel wird im Vakuum abdestilliert, der ölige Rückstand in Essigester gelöst, gekühlt, vom Ungelösten abfiltriert und nacheinander mit verdünnter $KHSO_4$-Lösung, gesättigter $NaHCO_3$-Lösung, Wasser und gesättigter NaCl-Lösung gewaschen. Nach Trocknen über $MgSO_4$ wird das Lösungsmittel abdestilliert und 4,3 g (= 94,8 % der Theorie) eines farblosen, zähen Öls erhalten.

Analog werden die R S S- und R,S S S-analogen Diastereomeren erhalten.

Beispiel 9

N-[N-(3-Phenyl-2-R-mercaptopropanoyl)-S-alanyl]-S-prolin

Analog Beispiel 8 erhält man beim Einsatz von N-[N-(3-Phenyl-2-S-brompropanoyl)-S-alanyl]-S-prolin-tert. butylester die diastereomere Titelverbindung N-[N-(3-Phenyl-2-R-mercaptopropanoyl)-S-alanyl]-S-prolin.
Rf = 0,54 (Chloroform, Methanol, Eisessig = 90:10:5, Silicagel).

Beispiel 10

N-[N-(3-Phenyl-2-R,S-mercaptopropanoyl)-S-alanyl]-S-prolin

Analog Beispiel 8 erhält man beim Einsatz von N-[N-(3-Phenyl-2-R,S-brompropanoyl)-S-alanyl]-S-prolin-tert. butylester die Titelverbindung N-[N-(3-Phenyl-2-R,S-mercaptopropanoyl)-S-alanyl]-S-prolin.

Beispiel 11

N-[N-(Mercaptoacetyl)-S-alanyl]-S-prolin

Analog Bespiel 8 erhält man beim Einsatz von N-[N-Bromacetyl)-S-alanyl]-S-prolin-tert. butylester die Titelverbindung N-[N-(Mercaptoacetyl)-S-alanyl-S-prolin.
Rf = 0,35 (Chloroform, Methanol, Eisessig = 90:10:5, Silicagel).

Beispiel 12

N-[N-(2-Phenyl-2-R,S-mercaptoacetyl)-S-alanin]-S-prolin

Analog Beispiel 8 erhält man beim Einsatz von N-[N-(2-Phenyl-2-R,S-bromacetyl)-S-alanin]-S-prolin-tert. butylester die Titelverbindung N-[N-(2-Phenyl-2-R,S-mercaptoacetyl)-S-analnin]-S-prolin.
Rf = 0,48 (Chloroform, Methanol, Eisessig = 90:10:5, Silicagel).

Beispiel 13

N-[N-(4-Phenyl-2-R,S-mercaptobutanoyl)-S-alanin]-S-prolin

Analog Beispiel 8 erhält man beim Einsatz von N-[N-(4-Phenyl-2-R,S-brombutanoyl)-S-alanin]-S-prolin die Titelverbindung N-[N-(4-Phenyl-2-R,S-mercaptobutanoyl)-S-alanin]-S-prolin.
Rf = 0,59 (Chloroform, Methanol, Eisessig = 90:10:5, Silicagel).

0230922

Beispiel 14

N,N'-[N,N'- 2,2'-dithiobis-(3-Phenylpropanoyl) -bis-S-alanyl]-bis-S-prolin

2,5 g (4,95 mMol) N,N'-[2,2'-S-dithiobis-(3-phenylpropanoyl)]-bis-S-alanin und 1,69 g (9,9 mMol) S-Prolin-tert. butylester werden in 50 ml wasserfreiem Dichlormethan gelöst und unter Rühren und Eiswasserkühlung mit 2,0 g (9,90 mMol) N,N'-Dicyclohexylcarbodiimid versetzt. Es wird noch 15 Minuten, dann über Nacht bei Raumtemperatur nachgerührt, abgesaugt und vom Lösungsmittel abdestilliert. Der Rückstand wird in Essigester gelöst, kaltgestellt, erneut abfiltriert und mit verdünnter $KHSO_4$-Lösung, gesättigter $NaHCO_3$-Lösung, Wasser und gesättigter NaCl-Lösung gewaschen. Nach Trocknen über $MgSO_4$ wird der Essigester im Vakuum abdestilliert und dabei 3,4 g eines erstarrten Schaumes erhalten. Der Rückstand wird über Kieselgel chromatographiert (Laufmittel: Dichlormethan, Methanol (95:5)).
2,6 g der Titelverbindung in der Bis-tert. butylesterform chromatographisch und NMR-spektroskopisch einheitlich werden erhalten.

Zur Verseifung des Diesters wird eine Mischung von 2,6 g (3,2 mMol) des obigen dimeren tert. Butylesters, 13 ml Anisol und 26 ml Trifluoressigsäure 2 Stunden lang bei Raumtemperatur gerührt und anschließend im Vakuum zur Trockne eingedampft. Der Rückstand (2,7 g) wird jeweils dreimal mit Aceton und Chloroform versetzt und erneut im Vakuum abdestilliert. Das Rohprodukt wird in Dichlormethan aufgenommen, mit gesättigter $NaHCO_3$-Lösung gewaschen, die wäßrige Phase mit konzentrierter HCl angesäuert und der kristalline Niederschlag abgesaugt, mit Wasser gewaschen und getrocknet. -

1,1 g (= 49 % der Theorie) der Titelverbindung werden chromatographisch und NMR-spektroskopisch rein erhalten; Fp. 178°C.
Rf = 0,62 (Chloroform, Methanol, Eisessig = 90:10:5, Silicagel).

Die Herstellung des R,S S S-analogen Dimeren erfolgt nach der gleichen Vorschrift; Fp. 174°C
Rf = 0,57 (Chloroform, Methanol, Eisessig = 90:10:5, Silicagel).

Die Ausgangsverbindungen werden wie folgt hergestellt:

N-(3-Phenyl-2-S-acetylmercaptopropanoyl)-S-alanin-methylester

Zu einer Lösung von 1,98 g (26 mMol) Thiolessigsäure in 70 ml wasserfreiem Ether werden unter Rühren, Kühlung auf 0°C und $N_2$-Atmosphäre 2,6 g (26 mMol) Triethylamin, gelöst in 20 ml wasserfreiem Ether, langsam zugetropft. Anschließend werden dieser Lösung 4,1 g (13 mMol) N-(3-Phenyl-2-R-brompropanoyl)-S-alanin-methylester (Herstellung s. Beispiel 1) in 50 ml wasserfreiem Ether tropfenweise zugesetzt und 90 Minuten am Rückfluß gekocht. Nach Abkühlen auf Raumtemperatur wird filtriert und das Lösungsmittel abdestilliert. Der ölige Rückstand (6,2 g) wird über Kieselgel mit n-Hexan, Essigester (2:1) chromatographiert und 3,9 g (96,9 % der Theorie) eines NMR-spektroskopisch einheitlichen, kristallinen Produkts als Titelverbindung erhalten.

N,N'-[2,2'-S-dithiobis-(3-phenylpropanoyl)]-bis-S-alanin

3,9 g (12,6 mMol) N-(3-Phenyl-2-S-acetylmercaptopropanoyl)-S-alanin-methylester werden in 40 ml Methanol gelöst und unter Rühren und Eiskühlung mit 37,8 ml (37,8 mMol) 1 n NaOH versetzt. Die Lösung wird 3 Stunden bei Raumtemperatur gerührt, dann mit einer 2 %igen methanolischen Jodlösung bis zur bleibenden Gelbfärbung versetzt. Anschließend wird mit verdünnter $Na_2S_2O_3$-Lösung entfärbt, vom Lösungsmittel im Vakuum abdestilliert, der Rückstand in Wasser gelöst und mit Dichlormethan gewaschen. Die wäßrige Phase wird im Vakuum zur Entfernung des Dichlormethans eingeengt und mit konzentrierter HCl angesäuert. Das ausgefallene Reaktionsprodukt wird mit Dichlormethan extrahiert, mit gesättigter NaCl-Lösung gewaschen, über $MgSO_4$ getrocknet und vom Lösungsmittel abdestilliert.
2,5 g (= 78,6 % der Theorie) der Titelverbindung können als erstarrter Schaum isoliert werden.
Rf = 0,5 (Chloroform, Methanol, Eisessig = 90:10:5, Silicagel).
Die Herstellung der R S S- und R,S S S-analogen Diastereomeren erfolgt nach dem gleichen Verfahren.

Beispiel 15

N-[N-(3-Phenyl-2-S-{4-chlor-3-sulfon-amidobenzoyl}-mercaptopropanoyl-S-alanyl]-S-prolin

Zu einer Lösung von 2,1 g (6 mMol) N-[N-(3-Phenyl-2-S-mercaptopropanoyl)-S-alanyl]-S-prolin in 100 ml Wasser und 1,008 g (12 mMol) $NaHCO_3$ werden 1,524 g (6 mMol) 4-Chlor-3-sulfamoylbenzoesäurechlorid gegeben und 2 Stunden bei Raumtemperatur weitergerührt. Nach 2-tägigem Stehen wird mit Dichlormethan gewaschen, die wäßrige Phase mit verdünnter HCl angesäuert und die ausgefallenen Kristalle abgesaugt.
1,4 g (= 82,1 % der Theorie) der Titelverbindung können als Festsubstanz isoliert werden.
Rf = 0,3 (Butanon, Aceton, Wasser = 60:6:10, Silicagel). Die NMR-spektroskopischen Daten bestätigen die Struktur und die Reinheit der Substanz.

Beispiel 16

N-[N-(3-Phenyl-2-S-mercaptopropanoyl)-S-alanyl]-S-prolyl-S-phenylalanin

Zu einer Lösung von 815 mg (10,7 mMol) Thiolessigsäure in 35 ml wasserfreiem Ether werden unter $N_2$-Atmosphäre, Eiskühlung und Rühren langsam 1,08 g (10,7 mMol) Triethylamin zugetropft. Anschließend wird eine Lösung von 3 g (5,37 mMol) N-[N-(3-Phenyl-2-R-brompropanoyl)-S-alanyl]-S-prolyl-S-phenylalaninmethylester in 20 ml wasserfreiem Dichlormethan tropfenweise zur obigen Lösung gegeben und 90 Minuten am Rückfluß gekocht. Nach dem Abkühlen wird abgesaugt und der Filterrückstand in Dichlormethan gelöst. Man wäscht mit gesättigter $NaHCO_3$-Lösung, verdünnter $KHSO_4$-Lösung, Wasser

und gesättigter NaCl-Lösung. Nach dem Trocknen über $MgSO_4$ wird das Lösungsmittel abdestilliert und 3,3 g eines festen Rückstandes erhalten, der nach Umkristallisation aus Essigester eine Ausbeute von 2,03 g (= 68 % der Theorie) ergab.
Der Methylester hat einen Fp. von 157 - 158°C und einen Rf-Wert von 0,13 (Essigester, n-Hexan = 2:1, Silicagel).

Zur Verseifung werden zu einer Suspension von 2 g (3,6 mMol) des obigen Mercaptoacyl-methylesters in 50 ml Methanol und 20 ml Wasser unter Rühren und einer $N_2$-Atmosphäre 6 ml 5 n NaOH gegeben und 1,5 Stunden bei Raumtemperatur gerührt. Man gibt weitere 2 ml 5 n NaOH zu und rührt nochmal 1,5 Stunden weiter. Das Methanol wird im Vakuum abrotiert, der Rückstand mit Wasser verdünnt und zweimal mit Dichlormethan gewaschen. Die wäßrige Phase wird mit verdünnter HCl-Lösung angesäuert und dreimal mti Dichlormethan extrahiert. Die organische Phase wird dann mit gesättigter NaCl-Lösung gewaschen, über $MgSO_4$ getrocknet und im Vakuum abdestilliert.
1,7 g (= 94,9 % der Theorie) der Titelverbindung werden als erstarrter Schaum erhalten, deren Struktur NMR-spektroskopisch bestätigt wurde.
Rf = 0,48 (Chloroform, Methanol, Eisessig = 90:10:5, Silicagel).
Nach dem gleich Verfahren werden die R,S S S- und R S S-analogen Diastereomeren erhalten.

Die Ausgangsverbindung wird wie folgt hergestellt:

N-[N-(3-Phenyl-2-R-brompropanoyl)-S-alanyl]-S-prolyl-S-phenylalanin

Zu einer Lösung von 2,8 g (7 mMol) N-[N-(3-Phenyl-2-R-brompropanoyl)-S-alanyl]-S-prolin,

1,5 g (7 mMol) S-Phenylalaninmethylester-hydrochlorid und 708 mg (7 mMol) Triethylamin in 50 ml wasserfreiem Dichlormethan werden unter Rühren bei 0°C 1,44 g (7 mMol) N,N'-Dicyclohexylcarbodiimid gegeben, 15 Minuten bei 0°C, dann über Nacht bei Raumtemperatur weitergerührt. Man filtriert, destilliert das Lösungsmittel im Vakuum ab und löst den Rückstand in Essigester. Dann wird die Lösung gekühlt, erneut filtriert und nacheinander gewaschen mit verdünnter $KHSO_4$-Lösung, gesättigter $NaHCO_3$-Lösung, Wasser, gesättigter NaCl-Lösung. Es wird über $MgSO_4$ getrocknet und das Lösungsmittel im Vakuum abdestilliert. Der Rückstand (4 g) wird aus wenig Essigester umkristallisiert;
Ausbeute: 3 g (76,7 % der Theorie)
Rf = 0,37 (Essigester, n-Hexan = 2:1, Silicagel).

Beispiel 17

N-[N-(3-Phenyl-2-S-mercaptopropanoyl)-S-alanyl]-S-prolyl-S-valin

Analog Beispiel 16 erhält man beim Einsatz von N-[N-(3-Phenyl-2-R-brompropanoyl)-S-alanyl]-S-prolyl-S-valin die Titelverbindung.
Rf = 0,48 (Chloroform, Methanol, Eisessig = 90:10:5, Silicagel).

0230924

Beispiel 18

N-[N-(3-Phenyl-2-S-mercaptopropanoyl)-S-alanyl]-S-prolyl-S-prolin

Analog Beispiel 16 erhält man beim Einsatz von N-[N-(3-Phenyl-2-R-brompropanoyl)-S-alanyl]-S-prolyl-S-prolin die Titelverbindung.
Rf = 0,26 (Essigsäureethylester, Silicagel).

Beispiel 19

N-[N-(3-Phenyl-2-S-mercaptopropanoyl)-S-alanyl]-S-thiazolidin-4-carbonsäure

Analog Beispiel 8 erhält man beim Einsatz von S-Thiazolidin-4-carbonsäure die Titelverbindung in Ausbeuten von 85,4 % der Theorie.
Fp. 144 - 146°C
Rf = 0,46 (Chloroform, Methanol, Eisessig = 90:10:5, Silicagel).
Elementaranalyse: $C_{16}H_{20}N_2O_4S_2$

| | C | H | N | S |
|---|---|---|---|---|
| Berechnet: | 52,25 | 5,47 | 7,60 | 17,40 |
| Gefunden: | 51,96 | 5,41 | 7,46 | 17,41 |

Die Struktur ist NMR-spektroskopisch bestätigt.

0230922

Beispiel 20

N-[N-(3-Phenyl-2-S-mercaptopropanoyl)-S-alanyl]-
4,5,6,7-tetrahydro-thieno[2,3-c]pyridin-7-carbonsäure

Analog Beispiel 8 erhält man beim Einsatz von 4,5,6,7-Tetrahydro-thieno[2,3-c]pyridin-7-carbonsäure in der R,S-, R- und S-Konfiguration die entsprechenden Diastereomeren der Titelverbindung. Rf-Wert für das S S R,S-Diastereomerengemisch: 0,18 (Chloroform, Methanol, Eisessig = 90:10:5, Silicagel).

Beispiel 21

N-[N-(3-Phenyl-2-S-mercaptopropanoyl)-S-alanyl]-
4,5,6,7-tetrahydro-thieno[3,2-c]pyridin-4-carbonsäure

Analog Beispiel 8 erhält man beim Einsatz von 4,5,6,7-Tetrahydro-thieno[3,2-c]pyridin-4-carbonsäuren die entsprechenden Diastereomeren der Titelverbindung. Rf-Wert für das S S R,S-Diastereomerengemisch: 0,53 (Chloroform, Methanol, Eisessig = 90:10:5, Silicagel).

Beispiel 22

N-[N-(3-Phenyl-2-S-benzoylmercaptopropanoyl)-S-alanyl]-
S-prolin

Zu einer Lösung von 2,1 g (15,4 mMol) Thiolbenzoesäure in 50 ml wasserfreiem Ether werden bei 0°C, Stickstoffatomosphäre und Rühren langsam 1,56 g (15,4 mMol) Triethylamin zugesetzt. Anschließend tropft man eine Lösung von 3,5 g (7,7 mMol) N-[N-(3-Phenyl-2-R-brompropanoyl)-S-prolin-tert.butylester in 30 ml wasserfreiem Ether zu und kocht 90 Minuten unter Rückfluß. Nach Absaugen wird

die etherische Lösung mit verdünnter $KHSO_4$-Lösung, gesättigter $NHCO_3$-Lösung, Wasser und gesättigter NaCl-Lösung gewaschen, über $MgSO_4$ getrocknet und zur Trockne eingedampft. Der Rückstand (3,7 g) wird über Kieselgel chromatographiert (Elutionsmittel: Essigester, N-Hexan = 1:1) und 3,4 g (= 86,5 % der Theorie) des tert. Butylesters der Titelverbindung als erstarrter Schaum erhalten.

Rf = 0,27 (Essigester, n-Hexan = 1:1, Silicagel).

Zur Verseifung werden 3,4 g (6,6 mMol) des obigen tert.Butylesters in 34 ml Trifluoressigsäure und 17 ml Anisol gelöst und 2 Stunden bei Raumtemperatur gerührt. Im Vakuum wird die Reaktionslösung eingedampft und je dreimal mit Aceton und Chloroform gelöst und erneut im Vakuum einrotiert. Der Rückstand wird in Dichlormethan gelöst und mit Wasser und gesättigter NaCl-Lösung gewaschen. Nach Trocknen über $MgSO_4$ wird eingedampft und der Rückstand (3,1 g) über Kieselgel chromatographiert (Elutionsmittel: Dichlormethan, Methanol, Eisessig = 120:5:2).
2,2 g (= 73,3 % der Theorie) der Titelverbindung werden als farbloser, erstarrter Schaum erhalten.
Rf = 0,25 (Dichlormethan, Methanol, Eisessig = 120:5:2, Silicagel).

Beispiel 23

N-[N-(3-Phenyl-2-S-pivaloylmercaptopropanoyl)-S-alanyl-S-prolin

Gearbeitet wird wie im vorangehenden Beispiel beschrieben. Anstelle der Thiolbenzoesäure steht Thiolpivalinsäure. Die Umsetzung von 3,17 g (7 mMol) N-[N-(3-Phenyl-2-R-brompropanoyl)-S-alanyl]-S-prolin-tert.butylester und 1,65 g (14 mMol) Thiolpivalinsäure ergaben 3,3 g (= 96 % der Theorie) des tert.Butylesters der Titelverbindung. Rf = 0,34 (Essigester, n-Hexan = 1:1, Silicagel). Seine Verseifung mit Trifluoressigsäureführte zur Titelverbindung in einer Ausbeute von 2,4 g (= 82,4 % der Theorie).
Rf = 0,3 (Dichlormethan, Methanol, Eisessig = 120:6:2, Silicagel).

Beispiel 24

N-[N-(3-Phenyl-2-S-acetylmercaptopropanoyl)-S-alanyl]-S-prolin-pivaloyloxymethylester

2,3 g (5,8 mMol) N-[N-(3-Phenyl-2-S-acetylmercaptopropanoyl)-S-alanyl]-S-prolin (Zwischenprodukt aus Beispiel 8) werden in 40 ml wasserfreiem Aceton gelöst und unter Rühren und Stickstoffatmosphäre nacheinander 580 mg (5,8 mMol) $KHCO_3$, 161 mg (0,97 mMol) Kaliumjodid und 873 mg (5,8 mMol) Chlormethylpivalat zugesetzt. Die Mischung wird 3 Stunden am Rückfluß gekocht und nach Abkühlen abgesaugt. Das Filtrat wird im Vakuum eingeengt, der Rückstand in Essigester aufgenommen und der Reihe nach gewaschen mit Wasser, gesättigter $NaHCO_3$-Lösung, Wasser und gesättigter NaCl-Lösung. Man trocknet über $MgSO_4$ und destilliert dasLösungsmittel im Vakuum ab. Der ölige Rückstand (2,7 g) wird über Kieselgel chromatographiert. (Laufmittel: Essigester, n-Hexan = 2:1) und dabei 2,2 g (= 75 % der Theorie) der Titelverbindung als Öl erhalten.
Rf = 0,42 (Essigester, n-Hexan = 2:1, Silicagel).

Beispiel 25

N-[N-(3-Phenyl-2-S-benzoylmercaptopropanoyl)-S-alanyl]-S-prolin-pivaloyloxymethylester

Analog Beispiel 24 erhält man beim Einsatz von N-[N-(3-Phenyl-2-S-benzoylmercaptopropanoyl)-S-alanyl-S-prolin die Titelverbindung.
Rf = 0,43 (Essigester, n-Hexan = 1:1, Silicagel).

Beispiel 26

N-[N-(3-Phenyl-2-S-pivaloylmercaptopropanoyl)-S-alanyl]-S-prolin-pivaloyloxymethylester

Analog Beispiel 24 erhaält man beim Einsatz von N-[N-(3-Phenyl-2-S-pivaloylmercaptopropanoyl)-S-alanyl]-S-prolin die Titelverbindung.
Rf = 0,45 (Essigester, n-Hexan = 2:1, Silicagel).

Beispiel 27

N-[N-(3-Phenyl-2-S-acetylmercaptopropanoyl)-S-alanyl]-N-cyclopentylglycin

6 g (20 mMol) N-(3-Phenyl-2-R-brompropanoyl)-S-alanin und 4 g (20 mMol) N-Cyclopentylglycin-tert.butylester werden in 100 ml Dichlormethan gelöst und unter Rühren und Kühlen (0°C) mit 4,1 g (20 mMol) N,N'-Dicyclohexyl-carbodiimid versetzt und 15 Minuten bei dieser Temperatur, dann über Nacht bei Raumtemperatur weitergerührt. Nach Absaugen des Dicyclohexylharnstoffs wird das Lösungsmittel im Vakuum abdestilliert, der ölige Rückstand in Essigester gelöst und einige Zeit in der Kälte stehen gelassen. Nach erneutem Absaugen wird das Filtrat mit verdünnter $KHSO_4$-Lösung, gesättigter $NaHCO_3$-Lösung, Wasser und gesättigter NaCl-Lösung gewaschen und nach Trocknen über $MgSO_4$ im Vakuum abdestilliert. Der ölige Rückstand (6,7 g) wird über Kieselgel chromatographiert (Laufmittel: Essigester, n-Hexan = 1:2).
Ausbeute 4,3 g (= 44,6 % der Theorie) Brom-tert. Butylester als farbloses Öl werden erhalten.
Rf = 0,28 (Essigester, n-Hexan = 1:2, Silicagel).

Der Austausch des Broms gegen Thiolessigsäure und die anschließende Verseifung des tert. Butylesters zur Titelverbindung erfolgt in der im Beispiel 8 beschriebenen Weise.
Rf = 0,55 (Chloroform, Methanol, Eisessig = 90:10:5)

Beispiel 28

N-[N-(3-Phenyl-2-S-methylaminothiocarbonyl-mercapto-propanoyl)-S-alanyl]-S-prolin

Eine Lösung von 1,73 g (4,96 mMol) N-[N-(3-Phenyl-2-S-mercaptopropanoyl)-S-alanyl]-S-prolin in 10 ml 0,5 n NaOH und 25 ml Pyridin wird unter Stickstoffatmosphäre mit 0,4 g (5,47 mMol) Methylisothiocyanat versetzt und 2 Stunden auf 40°C erwärmt. Danach wird im Vakuum zur Trockne eingedampft, der Rückstand mit Wasser aufgeschlämmtund mit konzentrierter HCl angesäuert. Die wäßrige Phase wird mit Essigester extrahiert, mit NaCl-Lösung gewaschen und über $MgSO_4$ getrocknet. Der Essigesterextrakt wird zur Trockne eingedampft und dabei 1,5 g (71,4 % der Theorie) der Titelverbindung als erstarrter Schaum erhalten, der über Kieselgel chromatographisch gereinigt wird. (Laufmittel: Toluol, Essigsäure = 75:25).
Rf = 0,47 (Chloroform, Methanol, Eisessig = 90:10:5, Silicagel).

Beispiel 29

N-[N-(3-Phenyl-2-S-ethylaminocarbonyl-mercapto-propanoyl)-S-alanyl]-S-prolin

Eine Lösung von 1,72 g (4,92 mMol) N-[N-(3-Phenyl-2-S-mercaptopropanoyl)-S-alanyl]-S-prolin in 5 ml 1 n

NaOH und 5 ml Pyridin wird mit 0,45 ml (5,7 mMol) Ethylisocyanat versetzt und 4 Stunden bei 40°C unter $N_2$-Atmosphäre gerührt. Danach wird im Vakuum zur Trockne eingedampft. Der Rückstand wird in Wasser aufgeschlämmt, mit 0,1 n HCl angesäuert und, wie im voranstehenden Beispiel beschrieben, weiterverarbeitet.
1,7 g (82,1 % der Theorie) der Titelverbindung werden als erstarrter Schaum erhalten.
Rf = 0,45 (Chloroform, Methanol, Eisessig = 90:10:5, Silicagel).

## Beispiel 30

### N-[$N^{\alpha}$-(3-Phenyl-2-S-mercaptopropanoyl)-S-lysyl]-thiazolidin-4-S-carbonsäure

3,5 g (7,6 mMol) $N^{\alpha}$-(3-Phenyl-2-R-brompropanoyl)-$N^{\varepsilon}$-tert.butoxycarbonyl-S-lysin und 1,4 g (7,6 mMol) Thiazolidin-4-S-carbonsäuremethylester-hydrochlorid werden in 70 ml wasserfreiem Dichlormethan gelöst und unter Rühren und Kühlen auf 0°C 769 mg (7,6 mMol) Triethylamin, dann 1,6 g (7,6 mMol) N,N'-Dicyclohexylcarbodiimid zugegeben. Man rührt bei Raumtemperatur ca. 17 Stunden lang, saugt vom ausgeschiedenen Dicyclohexylharnstoff ab und destilliert das Lösungsmittel ab. Der ölige Rückstand wird in Essigester gelöst, nach Kühlung erneut abfiltriert und die organische Phase nacheinander mit verdünnter $KHSO_4$-Lösung, gesättigter $NaHCO_3$-Lösung, Wasser und gesättigter NaCl-Lösung gewaschen. Nach Trocknen über $MgSO_4$ wird das Lösungsmittel im Vakuum abdestilliert. 4,1 g des rohen, öligen Reaktionsproduktes werden über Kieselgel mit Essigester/n-Hexan = 1:1 als Elutionsmittel chromatographisch gereinigt und dabei 2,9 g (= 68,8 % der Theorie)

N-[$N^\alpha$-(3-Phenyl-2-R-brompropanoyl)-Nε-tert.butoxy carbonyl-S-lysyl]-thiazolidin-4-S-carbonsäuremethylester in Form eines erstarrten Schaumes erhalten, der aus Ether kristallisierte.
Rf = 0,43 (Essigester, n-Hexan = 2:1, Silicagel).
Zum Austausch des Broms gegen die Acetylmercaptogruppe wird eine Lösung von 792 mg (10,4 mMol) Thiolessigsäure in 35 ml wasserfreiem Ether unter Stickstoff und Eiskühlung langsam mit 1,05 g (10,4 mMol) Triethylamin versetzt und anschließend tropfenweise unter Rühren zu einer Lösung von 2,9 g (5,2 mMol) der oben genanntenBromverbindung in 20 ml Essigester gegeben.

Man kocht danach noch 60 Minuten am Rückfluß, filtriert die vorher gekühlte Lösung und wäscht nacheinander mit verdünnter $KHSO_4$-Lösung, $NaHCO_3$-Lösung, Wasser und gesättigter NaCl-Lösung. Nach dem Trocknen über $MgSO_4$ wird das Lösungsmittel im Vakuum abdestilliert und als Rückstand 3,3 g eines gelblichen Öles erhalten. Die chromatrographische Reinigung erfolgt über 100 g Kieselgel mit Essigester/n-Hexan = 2:1 als Elutionsmittel; es werden so 1,9 g (64,1 % der Threorie) reiner N-[$N^\alpha$-(3-Phenyl-2-S-acetylmercaptopropanoyl)-$N^\varepsilon$-tert.butoxycarbonyl-S-lysyl]-thiazolidin-4-S-carbonsäuremethylester als erstarrter Schaum erhalten.
Rf = 0,36 (Essigester, n-Hexan = 2:1, Silicagel).

Zur Abspaltung der tert.Butoxycarbonyl-Schutzgruppe werden 1,9 g (3,33 mMol) der vorstehenden Acetylmercaptoverbindung mit 40 ml Trifluoressigsäure 3 Stunden bei Raumtemperatur gerührt, anschließend im

Vakuum zur Trockne eingedampft und zur Entfernung restlicher Trifluoressigsäure nacheinander je dreimal in Aceton und in Chloroform gelöst und erneut eingedampft. Es werden 2,7 g N-[$N^{\alpha}$-(3-Phenyl-2-S-acetylmercaptopropanoyl)-S-lysyl]-thiazolidin-4-S-carbonsäuremethylester in Form eines zähen Öles erhalten.
Rf = 0,23 (Chloroform, Methanol, Eisessig = 90:10:5, Silicagel).

Zur gleichzeitigen Verseifung des Methyl- und des Thiolessigsäureesters werden die 2,7 g (3,3 mMol) des vorstehenden Reaktionsproduktes in 20 ml Methanol und 40 ml Wasser gelöst und mit 4 ml 5 n Natronlauge versetzt. Nach 1,5-stündigem Rühren werden 2 ml 5 n Natronlauge bei Raumtemperatur zugesetzt und nochmals 1,5 Stunden weitergerührt. Das Methanol wird imVakuum abdestilliert, der Rückstand mit Wasser versetzt und mit Dichlormethan ausgeschüttelt.

Die wäßrige Phase wird mit verdünnter Salzsäure angesäuert, das sich ausscheidende zähe Öl im Scheidetrichter abgetrennt und in wenig Wasser gelöst, über 100 g Dowex 50 W x 4 gereinigt (Elution mit 5%igem wäßrigem Pyridin).
700 mg (= 45,9 % der Threorie) N-[$N^{\alpha}$-(3-Phenyl-2-S-mercaptopropanoyl)-S-lysyl]-thiazolidin-4-S-carbonsäure werden NMR-spektroskopisch rein erhalten.
Rf = 0,52 (Essigester, Butanol, Wasser, Essigsäure = 1:1:1:1, Silicagel).

<u>Herstellung der Ausgangsverbindungen:</u>

<u>$N^{\alpha}$-(3-Phenyl-2-R-brompropanoyl)-$N^{\varepsilon}$-tert.butoxy-carbonyl-S-lysin</u>

3,25 g (14,2 mMol) 3-Phenyl-2-R-brompropionsäure und 3,7 g (14,2 mMol)

$N^{\varepsilon}$-tert.Butoxycarbonyl-S-lysinmethanester werden in 70 ml wasserfreiem Dichlormethan gelöst, unter Rühren und Kühlen (0°C) 2,9 g (14,2 mMol) N,N'-Dicyclohexylcarbodiimid zugegeben und über Nacht bei Raumtemperatur weitergerührt. Man saugt vom ausgeschiedenen Dicyclohexylharnstoff ab, entfernt das Lösungsmittel durch Destillation und nimmt den Rückstand in Essigester auf. Nach Kühlen und erneuter Filtration wird die organische Phase nacheinander mit verdünnter $KHSO_4$-Lösung, gesättigter $NaHCO_3$-Lösung, Wasser und gesättigter NaCl-Lösung gewaschen, über $MgSO_4$ getrocknet und im Vakuum zur Trockne eingedampft. Der kristalline Rückstand (6,2 g) wird aus Essigester und n-Hexan umkristallisiert und dabei 4,8 g (= 71,1 % der Threorie) des Methylesters der Titelverbindung erhalten; Fp. 97-98°C. Rf = 0,49 (Essigester, n-Hexan = 2:1, Kieselgel),
Rf = 0,7 (Chloroform, Methanol, Eisessig = 90:10:5, Silicagel).

Zur Verseifung des voranstehenden Methylesters werden 4,8 g (10,2 mMol) des $N^{\alpha}$-(3-Phenyl-2-R-brom-propanoyl)-$N^{\varepsilon}$-butoxycarbonyl-S-lysin-methylesters in 50 ml Methanol gelöst und unter Rühren und Eiswasserkühlung 11 ml 1 n Natronlauge (11 mMol) zugegeben. Nach dreistündigem Rühren bei Raumtemperatur wird das Methanol im Vakuum abgezogen, der wäßrige Rückstand mit Wasser verdünnt und die trübe Lösung mit 5%iger $KHSO_4$-Lösung angesäuert und mit Dichlormethan extrahiert. Das aus der Dichlormethanlösung ausgeschiedene, kristalline Produkt wird abgesaugt (2,32 g) und aus dem Filtrat noch weitere 2 g kristalline Masse erhalten. Beide Fraktionen werden vereinigt und aus Chloroform und wenig Methanol umkristallisiert. 3,1 g (= 66,4 % der Threorie) der Titelverbindung werden erhalten.
Fp. 178-179°C (Zers.)
Rf = 0,55 (Chloroform, Methanol, Eisessig = 90:10:5, Silicagel).

Beispiel 31

N-[$N^{\alpha}$-(3-Phenyl-2-S-acetylmercaptopropanoyl)-S-lysyl]-thiazolidin-4-S-carbonsäure

Das S-Acylderivat der Titelverbindung aus Beispiel 30 wird erhalten, wenn man, wie im Beispiel 30 beschrieben, anstelle des Thiazolidinmethylesters den entsprechenden Thiazolidin-4-S-carbonsäure-tert.-butylesters einsetzt und mit Trifluoressigsäure in einem Reaktionsschritt den tert.Butylester des Reaktionsproduktes verseift und gleichzeitig seine N-Schutzgruppe abspaltet.

Beispiel 32

N-[$N^{\alpha}$-(3-Phenyl-2-S-acetylmercapto)-S-lysyl]-S-prolin-pivaloyloxymethylester

3 g (6,6 mMol)
N-[$N^{\alpha}$-(3-Phenyl-2-S-acetylmercapto)-S-lysyl)-S prolin werden in Aceton gelöst, 660 mg (6,6 mMol) $KHCO_3$, 185 mg Kaliumjodid und 1,16 ml (8,06 mMol) Chlormethylpivalat zugegeben und 3 Stunden am Rückfluß erhitzt. Nach Abkühlung wird vom ausgefallenen KCl abfiltriert und das Filtrat eingeengt. Der Rückstand wird in Essigester aufgenommen, nacheinander mit Wasser, verdünnter $NaHCO_3$-Lösung, Wasser und gesättigter NaCl-Lösung gewaschen und über $MgSO_4$ getrocknet. Nach Abdestillieren des Essigester werden 2,1 g eines öligen Rückstandes erhalten, der über Kieselgel chromatographisch gereinigt wird (Elution: eingangs Essigester, n-Hexan = 2:1, dann Wechsel auf Essigester, Methanol = 9:1).
Es werden 0,8 g eines öligen Produktes erhalten, das NMR-spektroskopisch der Titelverbindung entspricht.
Rf = 0,36 (Essigester, Methanol = 9:1, Silicagel).

Beispiel 33

N-[$N^{\alpha}$-(3-Phenyl-2-S-acetylmercapto)-S-lysyl]-thiazolidin-4-S-carbonsäure-pivaloyloxymethylester

Der der Titelverbindung im Beispiel 32 analoge Thiazolidin-4-S-carbonsäure-pivaloyloxymethylester wird erhalten, wenn man anstelle der Prolinverbindung im Beispiel 32 die N-[$N^{\alpha}$-(3-Phenyl-2-S-acetylmercapto)-S-lysyl]-thiazolidin-4-S-carbonsäure einsetzt.

Beispiel 34

N,N'-[N,N'-{2,2'-S-Dithiobis-(3-phenylpropanoyl)}-bis-S-lysyl]-bis-S-prolin

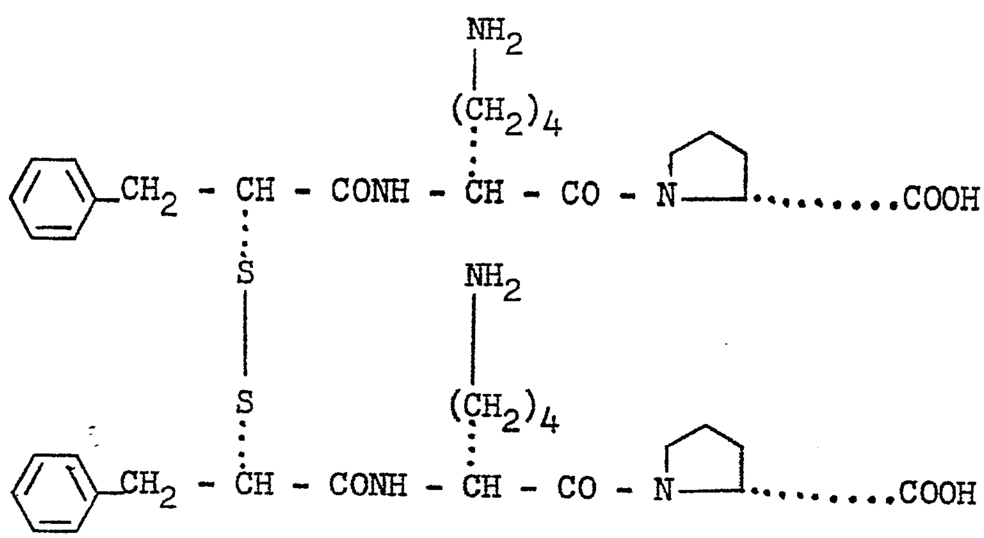

3,9 g (6,43 mMol) N-[$N^{\alpha}$-(3-Phenyl-2-S-acetylmercaptopropanoyl)-$N^{\varepsilon}$-tert.butoxycarbonyl-S-lysyl]-S-prolin-tert.butylester werden in 50 ml Methanol gelöst, mit 10 ml Wasser und 5,6 ml 5 n Natronlauge versetzt und 1,5 Stunden bei Raumtemperatur gerührt. Danach werden 2,8 ml 5 n Natronlauge zugegeben und weitere 1,5 Stunden gerührt. Man destilliert im Vakuum das Methanol ab, löst den Rückstand in 300 mlDichlormethan und schüttelt mit Wasser aus. Die

organische Phase wird nacheinander mit verdünnter $KHSO_4$-Lösung, Wasser und gesättigter NaCl-Lösung gewaschen, über $MgSO_4$ getrocknet und im Vakuum abdestilliert.
3,4 g (= 94 % der Theorie) der freien Mercaptoverbindung werden als Öl erhalten.

Zur Oxidation werden die 3,4 g (6 mMol) der vorstehenden entacetylierten Verbindung in 100 ml Dichlormethan gelöst, 0,8 g (8 mMol) $KHCO_3$ zugesetzt und eine 2%ige methanolische Jodlösung bei Raumtemperatur bis zur bleibenden Gelbfärbung zugetropft (Verbrauch ca. 25 ml). Man fügt ca. 50 ml Wasser zu und reduziert das überschüssige Jod mit einigen Tropfen einer $Na_2S_2O_3$-Lösung. Die organische Phase wird abgetrennt, mehrmals mit Wasser und gesättigter NaCl-Lösung gewaschen, über $MgSO_4$ getrocknet und im Vakuum zur Trockne eingeengt.
2,2 g (= 64,9 % der Threorie) der dimeren Bisverbindung werden als erstarrter Schaum erhalten.

Zur Verseifung der Ester- und $N^{\varepsilon}$-Schutzgruppe wird die Bisverbindung (2,2 g = 1,97 mMol) mit 60 ml Trifluoressigsäure übergossen und über Nacht bei Raumtemperatur gerührt. Man destilliert die Trifluoressigsäure im Vakuum ab, löst in verdünnter $NaHCO_3$-Lösung und schüttelt mit Essigester aus. Danach wird die wäßrige Phase mit verdünnter Salzsäure angesäuert und im Vakuum eingeengt. Der weiße, feste Rückstand wird drei- bis viermal mit Methanol ausgezogen, vom Ungelösten abfiltriert und das Lösungsmittel im Vakuum zur Trockne eingedampft. Als Ausbeute werden 1,6 g (= 100 % der Theorie) der Titelverbindung als erstarrter Schaum erhalten.
Rf = 0,2 (Butanol, Essigsäure, Wasser = 3:1:1, Silicagel).

Beispiel 35

N,N'-[N,N'-{2,2'-S-Dithiobis-(3-phenylpropanoyl)}-bis-S-lysyl]-bis-S-prolin-pivaloyloxymethylester

Ausgangsprodukt ist die Titelverbindung aus Beispiel 34. Die Veresterung zur Titelverbindung erfolgt analog der im Beispiel 32 geschilderten Vorgehensweise.

Beispiel 36

N,N-[N,N'-{2,2'-S-Dithiobis-(3-phenylpropanoyl)}-bis-S-lysyl]-bis-thiazolidin-4-S-carbonsäure

Ausgangsverbindung ist der N-[$N^{\alpha}$-(3-Phenyl-2-S-acetylmercaptopropanoyl)-$N^{\varepsilon}$-tert.butoxycarbonyl-S-lysyl]-thiazolidin-4-S-carbonsäure-tert.butylester. Die Dimerisierung und Schutzgruppenabspaltung zur Titelverbindung erfolgt in der im Beispiel 34 beschriebenen Weise.

Beispiel 37

N,N-[N,N'-{2,2'-S-Dithiobis-(3-phenylpropanoyl)}-bis-S-lysyl]-bis-thiazolidin-4-S-carbonsäure-pivaloyloxymethylester

Ausgangsverbindung ist die Titelverbindung aus Beispiel 36. Die Veresterung zur Titelverbindung erfolgt analog der im Beispiel 32 geschilderten Weise.

NMR-Daten für mehrere erfindungsgemäße Verbindungen wie folgt:

Beispiel 1 Titelverbindung

$^1H$-NMR Daten ($CDCl_3$)
$\delta$ = 1,20, s, 9H, $C(CH_3)_3$
1,34, d, J = 7Hz, 3H, $CH^3$-CH-
1,86, d, J = 10Hz, 1H, SH
1,82 - 2,42, m, 4H, Pro-$CH_2CH_2$-
2,91 - 3,81, m, 5H, Pro N-$CH_2$, Ø-$CH_2$CH-
4,49, m, 1H, Pro α CH
4,66, m, 1H, $CH_3$-CH-
5,75, m, 2H, O-$CH_2$-O
7,23, m, 6H, 5 Aryl-H, NH

Beispiel 8

$^1H$ NMR daten ($CD_3OD$)
$\delta$ = 1,31, d, J = 7Hz , 3H, $CH_3$-CH-
1,80 - 2,41, m, 4H, Pro - $CH_2CH_2$
3,16, m, 2H, Ø - $CH_2$-CH-
3,43 - 3,89, m, 3H, Pro-$NCH_2$, Ø-$CH_2$-CH-
4,41, m, 1H, Pro α CH
4,61, m, 1H, $CH_3$-CH-
7,24, m, 5H, Aryl-H
SH, NH, COOH, im Lösungsmittel-Blindpeak.

Beispiel 9

$^1H$ NMR Daten ($CDCl_3$)

$\delta$ = 1,15, d, J = 7Hz, 3H, $CH_3$-CH-
1,77 - 2,39, m, 4H, Pro $CH_2CH_2$-
2,05, d, J = 10Hz, SH
3,13, m, 2H, Ø-$CH_2$-CH-
3,43, m, 1H, Ø-$CH_2$-CH-
3,63, m, 2H, Pro-N-$CH_2$-
4,55, m, 1H, Pro $\alpha$ H
4,77, m, 1H, $CH_3$-CH-
7,25, m, 5H, Aryl-H
8,93, s, breit, COOH

Beispiel 11

$^1H$ NMR daten ($CD_3OD$)

$\delta$ = 1,35, d, 3H, J = 7Hz, $CH_3$-CH-
1,81 - 2,52, m, 4H, Pro-$CH_2CH_2$-
3,17, s, 2H, $SCH_2$-C(=O)
3,69, m, 2H, Pro N-$CH_2$
4,47, m, 1H, Pro $\alpha$-CH-
4,64, qu, 1H, J = 7Hz, $CH_3$-CH-
SH, NH and COOH im Lösungsmittel-Blindpeak

Beispiel 12

$^1$H-NMR ($CD_3OD$)

$\delta$ = 1,32, d, J = 7Hz, 3H, $CH_3$-CH-
1,77 - 2,45, m, 4H, Pro-$CH_2CH_2$-
3,65, m, 2H, Pro-N-$CH_2$
4,48, m, 1H, Pro $\alpha$ CH-
4,66, m, 1H, $CH_3$-CH-
4,82, s, 1H, Ø-CH-
7,24 - 7,59, m, 5H, Aryl-H
SH, NH, COOH im Lösungsmittel-Blindpeak

Beispiel 13

$\delta$ = 1,35, d, 3H, $CH_3$-CH-, J = 7Hz
2,08, m, 2H, Ø-$CH_2$-$CH_2$-
1,80 - 2,44, m, 4H, Pro-$CH_2$-$CH_2$-
2,72, m, 2H, Ø-$CH_2$-
3,26, m, 1H, Ø-$CH_2CH_2$-CH-
3,65, m, 2H, Pro-N$CH_2$-
4,50, m, 1H, Pro $\alpha$ CH-
4,77, m, 1H, $CH_3$-CH-
7,23, m, 5H, Aryl-H
7,50, m, 1H, NH,
8,96, s, 2H, SH COOH

Beispiel 14

$^{1}$H-NMR Daten ($CDCl_3$)

$\delta$ = 1,28, d, J = 7Hz, 6H, $CH_3$-CH-
1,66 - 2,51, m, 8H, Pro-$CH_2CH_2$-
3,06, m, 4H, Ø-$CH_2$-CH-
3,33 - 4,01, m, 6H, Pro-$NCH_2$, Ø-$CH_2$-CH-
4,42, m, 2H, Pro $\alpha$ CH
4,75, m, 2H, $CH_3$-CH-
6,75, s, 2H, COOH
7,21, m, 10H, Aryl-H
7,50, d, J = 8Hz, 2H, NH

Beispiel 18

$^{1}$H-NMR Daten ($CDCl_3$)

$\delta$ = 1,34, d, J = 7Hz, 3H, $CH_3$-CH-
1,97, d, J = 10Hz, 1H, SH
1,71 - 2,43, m, 8H, Pro $CH_2CH_2$
2,84 - 4,00, m, 7H, Pro $NCH_2$, Ø-$CH_2$-CH-
4,26 - 4,96, m, 3H, Pro $\alpha$ CH, $CH_3$-CH-
6,20, s, 2H, NH, COOH
7,26, m, 5H, Aryl-H

Beispiel 19

$^1$H-NMR Daten ($CDCl_3$)

δ = 1,35, d, J = 7Hz, 3H, $CH_3$-CH-
2,03, d, J = 10Hz, 1H, SH
3,20, m, 2H, Ø-$CH_2$-CH-
3,27, d, 2H, S-$CH_2$-CH-, J = 7Hz
3,50, m, 1H, Ø-$CH_2$-CH-
4,72, m, 2H, N-$CH_2$-S
4,77, m, 1H, $CH_3$-CH-
5,10, t, J = 7Hz, S$CH_2$-CH-
6,52, s, 1H, COOH
7,27, m, 5H, Aryl-N
7,78, d, J = 8Hz, 1H, NH

Beispiel 20

$^1$H-NMR Daten ($CD_3OD$)

δ = 1,33, d, J = 7Hz, 3H, $CH_3$-CH-
2,50 - 3,91, m, 7H, Ø-$CH_2$-CH-, -$CH_2$-$CH_2$-
4,99, m, 1H, $CH_3$-CH-
5,85, s, 1H, N-CH-C=O
6,84, d, 1H, thiophen 4H
7,34, d, 1H, " 5H
7,18, m, 5H, Aryl-H
SH, NH, COOH im Lösungsmittel-Blindpeak

Beispiel 21

$^1$H-NMR Daten ($CDCl_3$)

δ = 1,34, d, J = 7Hz, 3H, $\underline{CH_3}$-CH-

2,02, d, J = 9Hz, 1H, $\underline{SH}$

2,72 - 4,43, m, 7H, Ø-$\underline{CH_2}$-$\underline{CH}$-, -$CH_2$-$CH_2$-

5,00, m, 1H, $CH_3$-$\underline{CH}$-

5,91, s, 1H, $\underline{CH}$-COOH

7,23, m, 9H, Aryl-H, thiophen—H, $\underline{NH}$, COOH

Beispiel 22

$^1$H-NMR Daten ($CDCl_3$)

δ = 1,28, d, 3H, J = 7,0Hz, $\underline{CH_3}$-CH-

1,77 - 2,39, m, 4H, Pro-$CH_2CH_2$-

3,24, m, 2H, Ø-$\underline{CH_2}$-CH-

3,58, m, 2H, Pro-$NCH_2$

4,47, m, 1H, Ø-$CH_2$-$\underline{CH}$-

4,53, m, 1H, Pro α-$\underline{CH}$-

4,75, m, 1H, $CH_3$-$\underline{CH}$-

7,02 - 8,00, m, 10H, 2-aromatics

8,50, s, 1H, COO$\underline{H}$

Beispiel 23

$^1H$-NMR Daten ($CDCl_3$)

δ = 1,17, s, 9H, $C(CH_3)_3$
1,26, d, 3H, J = 7Hz, $CH_3$-CH-
1,82 - 2,27, m, 4H, Pro-$CH_2CH_2$-
3,14, m, 2H, Ø-$CH_2$-CH-
3,57, m, 2H, Pro N-$CH_2$-
4,19, m, 1H, Ø-$CH_2$-CH-
4,50, m, 1H, Pro α-CH-
4,73, m, 1H, $CH_3$-CH-
6,98, d, 1H, J = 8Hz, -NH
7,21, m, 5H, Aryl-H
8,30, s, 1H, COOH

Beispiel 24

$^1H$-NMR Daten ($CDCl_3$)

δ = 1,21, s, 9H, $C(CH_3)_3$
1,23, d, 3H, J = 7Hz, $CH_3$-CH-
1,77 - 2,27, m, 4H, Pro $CH_2CH_2$-
2,30, s, 3H, $CH_3$-C=O
3,15, m, 2H, Ø-$CH_2$-CH-
3,58, m, 2H, Pro-$NCH_2$
4,15, m, 1H, Ø-$CH_2$-CH-
4,48, m, 1H, Pro α CH-
4,67, m, 1H, $CH_3$-CH-
5,75, m, 2H, -O-$CH_2$-O-
6,92, d, 1H, J = 8Hz, NH
7,23, m, 5H, Aryl-H.

Beispiel 25

$^1$H-NMR Daten ($CDCl_3$)

δ = 1,19, s, 9H, $C(CH_3)_3$
1,31, d, 3H, $CH_3$-CH-, J = 7 Hz
1,80 - 2,34, m, 4H, Pro-$CH_2CH_2$-
3,26, m, 2H, Ø-$CH_2$-CH-
3,58, m, 2H, Pro-N $CH_2$-
4,48, m, 1H, Ø-$CH_2$-CH-
4,50, m, 1H, Pro α CH-
4,73, m, 1H, $CH_3$-CH-
7,00, d, 1H, J = 7Hz, NH-
5,76, m, 2H, O-$CH_2$-O
7,27, m, 5H, Aryl-H
7,34 - 8,02, m, 5H, Ø-C(=O)-H

Beispiel 26

$^1$H-NMR Daten ($CDCl_3$)

δ = 1,16, s, 9H, $C(CH_3)_3$
1,20, s, 9H, $C(CH_3)_3$
1,27, d, 3H, J = 7Hz, $CH_3$-CH-
1,82 - 2,32, m, 4H, Pro $CH_2CH_2$
3,16, m, 2H, Phe-$CH_2$-CH-
3,57, m, 2H, Pro N $CH_2$-
4,16, m, 1H, Ø-$CH_2$-CH-
4,48, m, 1H, Pro α CH-
4,71, m, 1H, $CH_3$-CH-
5,76, m, 2H, O-$CH_2$-O
6,82, d, 1H, J = 8 Hz, NH
7,22, m, 5H, Aryl-H

Beispiel 27

$^1$H-NMR Daten ($CDCl_3$)

δ = 1,31, d, 3H, J = 7Hz, $CH_3$-CH-
1,16 - 2,11, m, 8H, cyclopentyl-$CH_2$
2,28, s, 3H, $CH_3$-C=O
3,15, m, 2H, Ø-$CH_2$-CH-
2,92, m, 2H, N-$CH_2$-C=O
4,22, m, 1H, cyclopentyl-CH
4,26, m, 1H, Ø-$CH_2$-CH-
4,92, m, 1H, $CH_3$-CH-
5,61, s, 1H, COOH
7,18, d, 1H, J = 8Hz, NH
7,24, m, 5H, Aryl-H

Beispiel 28

$^1$H-NMR Daten ($CDCl_3$)

δ = 1,27, d, J = 7Hz, 3H, $CH_3$-CH-
1,73 - 2,41, m, 4H, Pro-$CH_2$-$CH_2$-
3,18, d, J = 4Hz, 3H, NH-$CH_3$
3,21, m, 2H, Ø-$CH_2$-CH-
3,57, m, 2H, Pro N-$CH_2$
4,48, m, 1H, Pro α-CH
4,65, m, 1H, $CH_3$-CH-
4,48, m, 1H, Ø-$CH_2$-CH-
7,24, m, 5H, Aryl-H
7,47, d, J = 7Hz, NH-CH-
8,48, qu, J = 4Hz, NH-$CH_3$
8,77, s, 1H, COOH

Beispiel 29

$^1$H-NMR Daten ($CDCl_3$)

$\delta$ = 1,11, t, J = 7Hz, 3H, $\underline{CH_3}$-$CH_2$
1,28, d, J = 7Hz, 3H, $\underline{CH_3}$-CH
1,77 - 2,41, m, 4H, Pro $CH_2CH_2$
3,15, m, 2H, ∅-$\underline{CH_2}$-CH-
3,23, m, 2H, N-$\underline{CH_2}$-$CH_3$
3,56, m, 2H, Pro-N-$CH_2$
4,19, m, ∅-$CH_2$-$\underline{CH}$-
4,52, m, Pro α CH
4,73, m, $CH_3$-$\underline{CH}$-
5,65, s, breit, 2xNH and COO$\underline{H}$
7,23, m, 5H, Aryl-H

Entsprechend den obigen Beispielen können auch die nachstehenden Verbindungen erhalten werden.

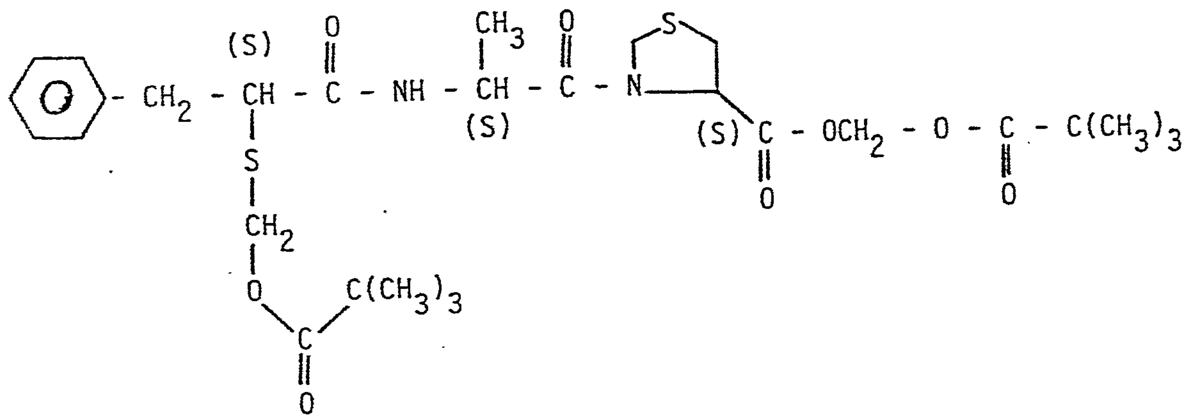

$^1$H-NMR Daten ($CDCl_3$)

$\delta$ = 1,15, s, 9H, $C(CH_3)_3$

1,22, s, 9H, $C-(CH_3)_3$

1,36, d, J = 7Hz, 3H, $\underline{CH_3}$-CH-

2,83 - 3,49, m, 4H, O-$\underline{CH_2}$-CH-, S-$\underline{CH_2}$-CH-

3,74, m, 1H, O-$CH_2$-$\underline{CH}$-

4,39 - 4,84, m, 3H, N-$CH_2$-S, $CH_3$-$\underline{CH}$-

5,07, m, 1H, S-$CH_2$-$\underline{CH}$-

5,76, m, 2H, O-$CH_2$-O

7,23, m, 6H, Aryl-H, $\underline{NH}$

Rf = 0,48 (Essigester, n-Hexan = 1:1)

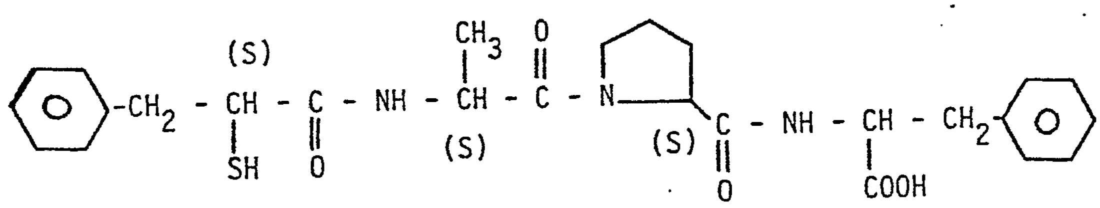

$^{1}$H-NMR Daten ($CDCl_3$)

$\delta$ = 1,05, d, J = 7Hz, 3H, $CH_3$-CH-

2,08, d, J = 10Hz, 1H, SH

1,59 - 2,25, m, 4H, Pro-$CH_2CH_2$

2,84 - 3,93, m, 7H, Pro-$NCH_2$, Ø-$CH_2$-CH-; Ø-$CH_2$-CH-COOH

4,46 - 4,99, m, 3H, Pro α CH, $CH_3$-CH-, Ø-$CH_2$-CH-COOH

7,24, m, 12H, Aryl-H, NH, COOH

7,75, d, J = 8Hz, NH

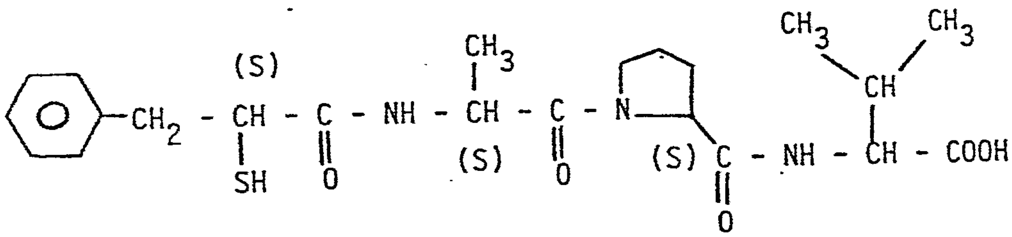

$^1$H-NMR Daten ($CDCl_3$)

$\delta$ = 0,86, d, J = 7Hz, 6H, Isobutyl-$CH_3$
1,32, d, J = 7Hz, 3H, $CH_3$-CH-
2,09, d, J = 10Hz, 1H, SH
1,71 - 2,70, m, 5H, Pro-$CH_2CH_2$, Isobutyl-CH-$(CH_3)_2$
3,14, m, 2H, Ø-$CH_2$-CH-
3,41 - 4,00, m, 3H, Pro-$NCH_2$, Ø-$CH_2$-CH-
4,34 - 4,99, m, 3H, Pro, α CH, $CH_3$-CH-, Isobutyl-CH
6,58, s, 1H, COOH
7,22, m, 5H, Aryl-H
7,53, d, J = 10Hz, 1H, NH-CH-$CH_3$
7,75, d, J = 8Hz, 1H, NH-Isobutyl

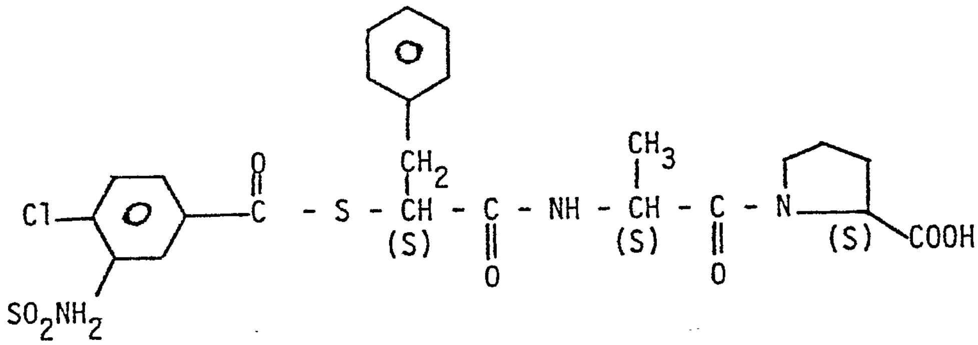

$^1$H-NMR Daten ($CD_3OD$)

$\delta$ = 1,30, d, J = 7Hz, 3H, $CH_3$-CH-

1,78 - 2,40, m, 4H, Pro $CH_2CH_2$

2,90 - 3,42, m, 2H, Ø-$CH_2$-CH-

3,56, m, 2H, Pro N-$CH_2$

4,27 - 4,84, m, 3H, Pro α CH, Ø-$CH_2$-CH-, $CH_3$-CH-

7,27, m, 5H, Aryl-H

7,73, d, J = 8Hz, 1H, ortho-Aryl-H

8,08, dd, J = 8Hz, 3Hz, 1H, ortho/meta Aryl-H

8,57, d, J = 3Hz, 1H, meta-Aryl-H

$NH_2$, NH, COOH im Lösungsmittel-Blindpeak

0230922

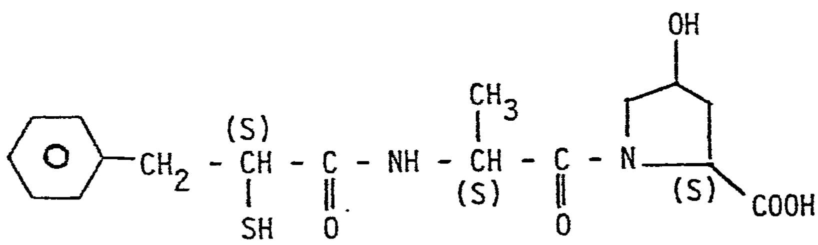

$^1$H-NMR Daten ($CDCl_3$)

δ = 1,33, d, J = 7Hz, 3H, $\underline{CH_3}$-CH-

2,02, d, J = 10Hz, 1H, S$\underline{H}$

2,00 - 2,50, m, 2H, Pro β-$CH_2$-

2,84 - 3,66, m, 3H, ~~Ø~~-$\underline{CH_2}$-$\underline{CH}$-

3,48 - 3,89, m, 2H, Pro-N-$CH_2$

4,50, m, 2H, Pro α-CH, $\underline{CH}$-OH

5,40, s, breit, O$\underline{H}$, COOH

7,24, m, 5H, Aryl-H

7,46, d, J = 8Hz, 1H, N$\underline{H}$

Rf = 0,23 (Chloroform, Methanol, Eisessig = 90:10:5)

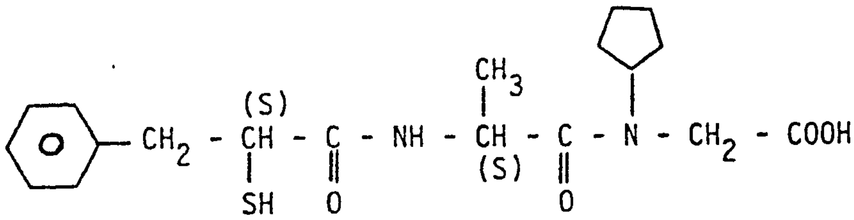

$^1$H-NMR Daten ($CDCl_3$)

δ = 1,34, d, 3H, J = 7Hz, $\underline{CH_3}$-CH-

1,34 - 1,99, m, 8H, cyclopentyl-$CH_2$-

2,00, d, J = 10Hz, S$\underline{H}$

3,16, m, 2H, Ø-$\underline{CH_2}$-CH-

3,53, m, 1H, Ø-$CH_2$-$\underline{CH}$-

3,92, m, 2H, N-$CH_2$-C=O

4,28, m, 1H, cyclopentyl-C$\underline{H}$

4,99, m, 1H, $CH_3$-$\underline{CH}$-

5,91, s, 1H, COO$\underline{H}$

7,24, m, 5H, Aryl-H

7,46, d, 1H, J = 8Hz, $\underline{NH}$

Rf = 0,63 (Chloroform, Methanol, Eisessig = 90:10:5)

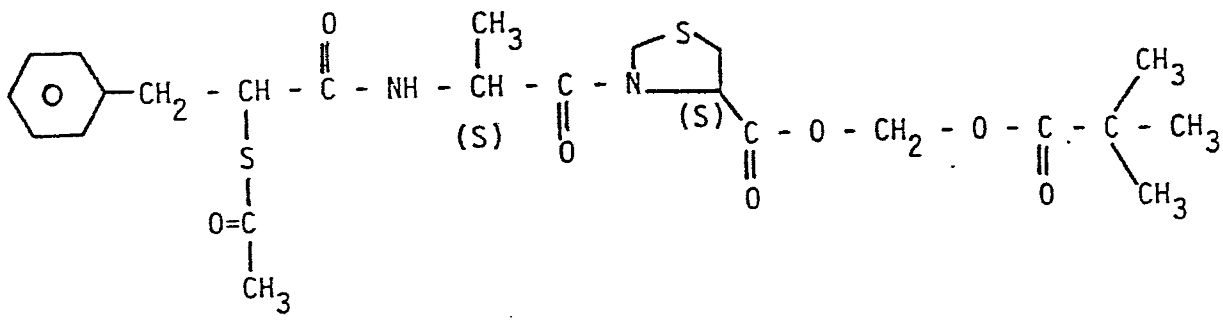

$^1$H-NMR Daten ($CDCl_3$)

$\delta$ = 1,19, s, 9H, $C(CH_3)_3$
1,32, d, J = 7Hz, 3H, $CH_3$-CH-
2,30, s, 3H, $CH_3$-C=O
3,11, m, 2H, Ø-$CH_2$-CH-
3,22, m, 2H, S-$CH_2$-CH-
4,12, m, 1H, Ø-$CH_2$-CH-
4,59, m, 2H, N-$CH_2$-S-
4,64, m, 1H, $CH_3$-CH-
5,07, m, 1H, N-CH-C=O
5,74, m, 2H, O-$CH_2$-O
6,86, d, J = 8Hz, 1H, NH
7,18, m, 5H, Aryl-H

Rf = 0,35 (Essigester, n-Hexane = 1:1)

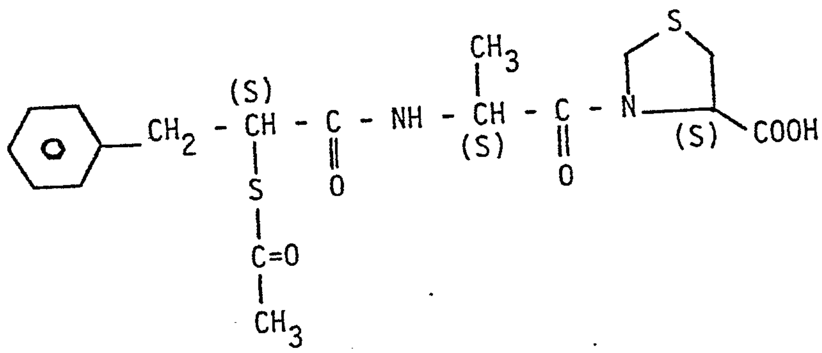

$^1$H-NMR Daten ($CDCl_3$)

$\delta$ = 1,32, d, 3H, J = 7Hz, $\underline{CH_3}$-CH-

2,30, s, 3H, $CH_3$-C=O

3,14, m, 2H, Ø-$\underline{CH_2}$-CH-

3,25, d, 2H, J = 6Hz, S$\underline{CH_2}$-CH-

4,25, m, 1H, Ø-$CH_2$-$\underline{CH}$-

4,65, m, 2H, N-$\underline{CH_2}$-S

4,78, m, 1H, $CH_3$-$\underline{CH}$-

5,07, t, 1H, J = 6 Hz, N-$\underline{CH}$-COO

7,16, d, 1H, J = 8Hz, NH

7,20, m, 5H, Aryl-H

8,48, s, 1H, COO$\underline{H}$

Rf= 0,47 (Chloroform, Methanol, Eisessig = 90:10:5)

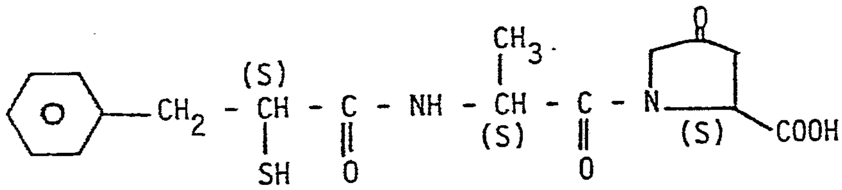

$^1$H-NMR Daten ($CDCl_3$)

$\delta$ = 1,30, d, J = 7Hz, 3H, $\underline{CH_3}$-CH-

2,09, d, J = 10Hz, 1H, S$\underline{H}$

2,48 - 3,81, m, 5H, Ø-$\underline{CH_2}$-$\underline{CH}$-, $\underline{CH_2}$-C=O

4,13, m, 2H, N-$\underline{CH_2}$

4,64, m, 1H, $CH_3$-$\underline{CH}$-

4,99, m, 1H, >$\underline{CH}$-COOH

7,24, m, 5H, Aryl-H

7,57, d, J = 8Hz, 1H, N$\underline{H}$

9,69, s, 1H, COO$\underline{H}$

Rf = 0,34 (Chloroform, Methanol, Eisessig = 120:5:2)

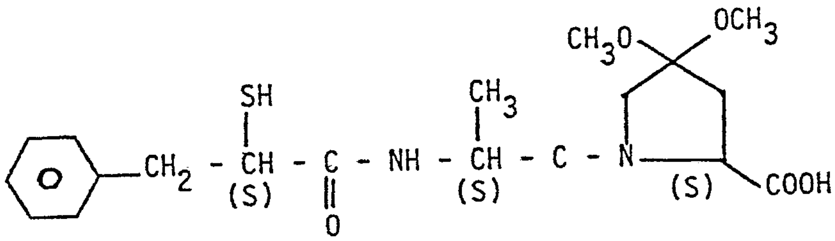

$^1$H-NMR Daten ($CDCl_3$)

$\delta$ = 1,31, d, 3H, J = 7Hz, $\underline{CH_3}$-CH-
2,05, d, 1H, J = 10Hz, S$\underline{H}$
2,35, d, 2H, J = 7Hz, Pro-$\underline{CH_2}$-CH-
3,19, m, 2H, ~~O~~-$CH_2$-CH-
3,27, s, 6H, 2 $OCH_3$
3,50, m, 1H, ~~O~~-$CH_2$-CH-
3,68, m, 2H, Pro-N-$CH_2$
4,59, m, 1H, Pro $\alpha$-CH-
4,70, m, 1H, $CH_3$-$\underline{CH}$-
5,95, s, breit, 2H, $\underline{NH}$ und COO$\underline{H}$
7,23, m, 5H, Aryl-H

Rf = 0,49 (Chloroform, Methanol, Eisessig = 90:10:5)

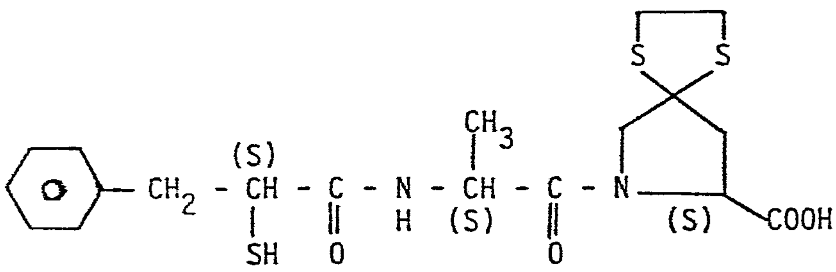

$^1$H-NMR Daten ($CDCl_3$)

$\delta$ = 1,30, d, J = 7Hz, 3H, $CH_3$-CH-
2,02, d, J = 10Hz, 1H, SH
2,67, m, 2H, Pro-$CH_2$-CH-
3,15, m, 2H, ø-$CH_2$-CH-
3,36, s, 4H, -S$CH_2CH_2$-S-
3,55, m, 1H, ø-$CH_2$-CH-
4,00, m, 2H, Pro-N-$CH_2$
4,64, m, 2H, $CH_3$-CH-, Pro $\alpha$-CH-
7,23, m, 5H, Aryl-H
7,41, d, J = 8Hz, 1H, NH
7,56, s, 1H, COOH

Rf = 0,5 (Chloroform, Methanol, Eisessig 90:10:5)

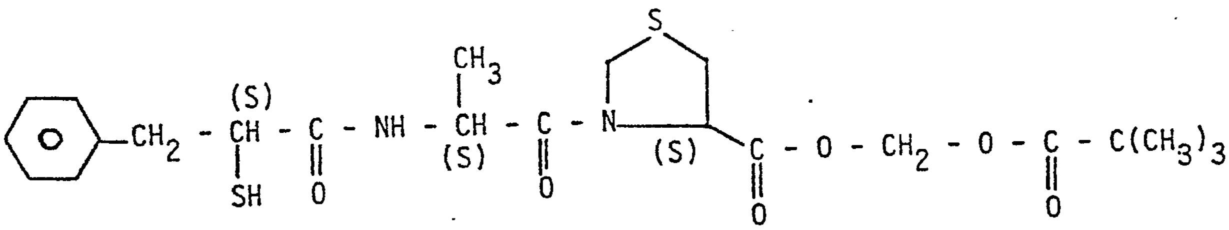

$^1$H-NMR Daten ($CDCl_3$)

δ = 1,18, s, 9H, $C(CH_3)_3$
1,34, d, J = 7Hz, 3H, $CH_3$-CH-
1,99, d, J = 10Hz, 1H, SH
2,93 - 3,77, m, 5H, Ø-$CH_2$-CH-, S$CH_2$-CH-
4,41 - 4,94, m, 3H, N-$CH_2$-S, $CH_3$-CH-
5,07, m, 1H, S-$CH_2$-CH-
5,77, m, 2H, O$CH_2$-O
7,07, d, J = 9Hz, 1H, NH
7,23, m, 5H, Aryl-H

Rf = 0,33 (Eisessig, n-Hexan = 1:1)

N-/N-3-Phenyl-2-S-pivaloyloxymethylmercaptopropanoyl)-S-alanyl7-S-thiazolidin-4-carbonsäure

$^1$H-NMR Daten ($CDCl_3$)

δ = 1,16, s, 9H, $C(CH_3)_3$
1,21, s, 9H, $C(CH_3)_3$
1,33, d, J = 7Hz, 3H, $CH_3$-CH-
1,78 - 2,43, m, 4H, Pro-$CH_2CH_2$
3,14, m, 2H, Ø-$CH_2$-CH-
3,34 - 3,89, m, 3H, Ø-$CH_2$-CH-, Pro-N$CH_2$
4,36 - 4,89, m, 2H, Pro α CH, $CH_3$-CH-
5,75, m, 2H, O-$CH_2$-O
7,14, d, J = 8Hz, 1H, NH
7,23, m, 5H, Aryl-H

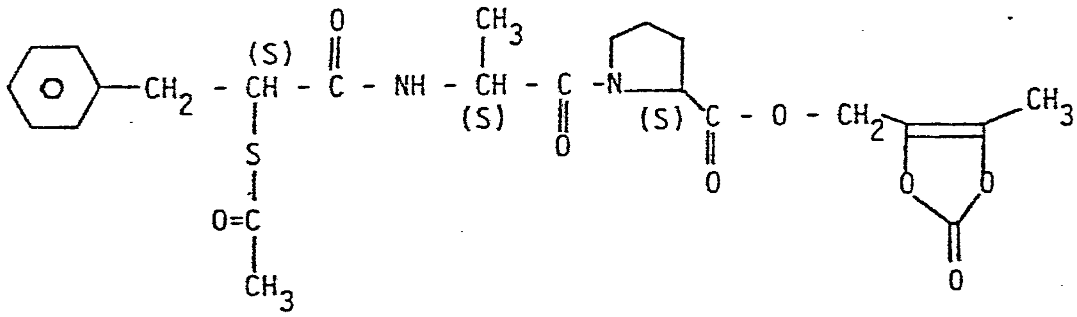

$^1$H-NMR Daten ($CDCl_3$)

δ = 1,29, d, (3H), J = 7Hz, $\underline{CH_3}$-CH-

1,76 - 2,31, m, 4H, Pro-$CH_2$-$CH_2$

2,16, s, 3H, $\underline{CH_3}$-C=

2,31, s, 3H, $CH_3$-C(=O)-

3,13, m, 2H, $\phi$-$CH_2$

3,58, m, 2H, Pro N-$CH_2$-

4,24, m, 1H, $\phi$-$CH_2$-$\underline{CH}$-

4,48, m, 1H, $CH_3$-$\underline{CH}$-

4,58, m, 1H, Pro α $\underline{CH}$-

4,78, m, 2H, O-$CH_2$-

6,87, d, 1H, J = 7,5Hz, $\underline{NH}$

7,23, m, 5H, Aryl-H

Rf = 0,17 (Essigester, Hexan = 2:1)

## Pharmazeutische Anwendungsbeispiele

a) Dragees

1 Drageekern enthält:

| | |
|---|---|
| Wirkstoff gemäß Anspruch 1 | 10,0 mg |
| Milchzucker | 60,0 mg |
| Maisstärke | 35,0 mg |
| Gelatine | 3,0 mg |
| Magnesiumstearat | 2,0 mg |
| | 110,0 mg |

Herstellung:

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragées werden mit Bienenwachs poliert.

b) Tabletten

| | |
|---|---|
| Wirkstoff gemäß Anspruch 1 | 10,0 mg |
| Milchzucker | 70,0 mg |
| Maisstärke | 50,0 mg |
| lösliche Stärke | 7,0 mg |
| Magnesiumstearat | 3,0 mg |
| | 140,0 mg |

Herstellung:
Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 230 mg Gewicht verpreßt, die je 100 mg Wirkstoff enthalten.

c) Injektionslösungen

| | | |
|---|---|---|
| Wirkstoff gemäß Anspruch 1 | | 5,0 mg |
| Äthanolamin | | 60,0 mg |
| Natriumchlorid | | 20,0 mg |
| destilliertes Wasser | ad | 2 ml |

Herstellung:
Der Wirkstoff und die Hilfsstoffe werden in einer ausreichenden Menge destilliertem Wasser gelöst und mit der erforderlichen Menge Wasser auf die gewünschte Konzentration gebracht. Die Lösung wird filtriert und unter aseptischen Bedingungen in 2 ml Ampullen abgefüllt. Die Ampullen werden sterilisiert und verschlossen. Jede Ampulle enthält 5 mg Wirkstoff.

d) Kapseln

| | |
|---|---|
| Wirkstoff gemäß Anspruch 1 | 10,0 mg |
| Milchzucker | 250,0 mg |
| Maisstärke | 40,0 mg |
| Talk | 10,0 mg |
| | 310,0 mg |

Herstellung:

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

e) Suppositorien

| | |
|---|---|
| Wirkstoff gemäß Anspruch 1 | 0,1 g |
| Kakaobutter (Fp. 36-37°C) | 1,6 g |
| Carnaubawachs | 0,1 g |
| | 1,8 g |

Herstellung:

Kakaobutter und Carnaubawachs werden geschmolzen, gründlich vermengt und auf 45°C abgekühlt. In diese Masse wird der feinpulverisierte Wirkstoff eingerührt. Anschließend wird die Mischung in leicht vorgekühlten Suppositorienformen geeigneter Größe gegossen und abkühlen lassen.

Literatur

1. M.F.R. Martin et. al. The Lancet 1984, S. 1325-27.

2. G. Trübestein et. al. Dr. med. Wochen 109 (1984), S. 857

3. G.S. Zübenko et. al. Am. J. Psychiatry 141:1, S. 110-111 (1984).

4. M.J. Antanaccio, Am. Rev. Pharmacol. Toxicol. 22, 57-58 (1982).

5. R. Kazin Türker, "Degradation of Endogenous Opiods: Its relevance in Human Pathology and Therapy; S. Ehrenpreis and F. Sicutevi, Raven Press, New York 1983, S. 142-159.

6. M. Fancin Hacci et. al. ibid S. 217-230.

PATENTANSPRÜCHE:

1. Die Verwendung einer Verbindung gemäß Formel I

$$R_1 - \underset{\displaystyle |}{\overset{SR_2}{|}}\!\!\!\!CH - CO - NH - \overset{R_3}{\overset{|}{CH}} - CO - R_4 \qquad I$$

$R_1$= H, Phenyl, Benzyl, Phenethyl

$R_2$= H, Acetyl, Benzoyl, Pivaloyl, Pivaloyloxymethyl, 3-Sulfonamido-4-chlorbenzoyl, 3-Sulfonamido-4-chlor-6-hydroxy-benzoyl, 3-Sulfonamido-4-chlor-5-[(furyl)-amino]-benzoyl, 2,3-Dichloro-4-(ß-phenyl-acryloyl)-phenoxy-acetyl, $(C_1-C_2)$alkylaminocarbonyl, $(C_1-C_2)$alkylaminothiocarbonyl oder den Rest der Formel

$$-S-\underset{\displaystyle R_1}{\underset{|}{CH}}-CO-NH-\overset{R_3}{\overset{|}{CH}}-CO-R_4 \qquad II$$

$R_3$= $-CH_3$ oder $-(CH_2)p-NHR$, worin p eine der Zahlen 1 - 4 und R H, Acetyl, Benzoyl, Pivaloyl oder tert. butoxycarbonyl bedeuten,

$R_4$= den Rest einer der α-Aminosäuren

$$\overset{R_5}{\overset{|}{HN}} - \overset{R_6}{\overset{\vdots}{C(H)}} - COR_7$$

oder

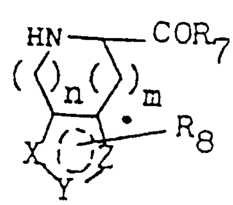

,

worin

$R_5$ = H, $(C_1-C_4)$Alkyl, gerade oder verzweigt $(C_5-C_7)$-Cycloalkyl, wobei ein Phenylring ankondensiert sein kann, Phenyl, Phenyl-$(C_1-C_4)$alkyl, einen Heterocyclus-$(C_1-C_4)$alkylrest, wobei der Heterocyclus aus einem 5- oder 6-Ring mit 1 bis 2 der Heteroatomen O,S und N besteht,

$R_6$ = H, $(C_1-C_4)$Alkyl, gerade oder verzweigt, Phenyl, Phenyl-$(C_1-C_4)$alkyl oder einen Heterocyclus-$(C_1-C_4)$alkylrest, wobei der Heterocyclus aus einem 5- oder 6-Ring mit 1 bis 2 der Heteroatome O,S und N besteht, oder

$R_5$ und $R_6$ zusammen mit dem N- und dem C-Atom einen 5-, 6- oder 7-gliedrigen Ring bilden, der gesättigt sein kann oder eine Doppelbindung enthalten kann, oder einen 4-, 5- oder 6-gliedrigen Ring, der ein oder zwei weitere der Heteroatome O,S und N enthält;

$R_7$ OH, $(C_1-C_4)$-ω-Hydroxyalkyl, $(C_1-C_4)$-Alkoxy, Phenyl-$(C_1-C_4)$alkoxy, $(C_1-C_4)$alkylamino, Di-$(C_1-C_4)$alkylamino, eine der Gruppen

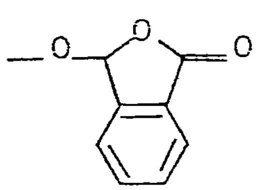

,

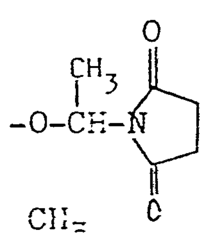

,

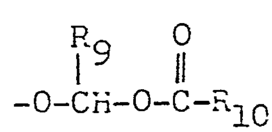

oder

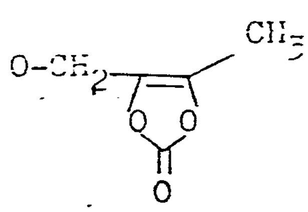

.

oder eine α-Aminosäure, die peptidartig mit der =CO Gruppe des Moleküls verknüpft ist;
m und n jeweils 0,1 oder 2, wobei die Summe aus m und n 1 oder 2 ist, bedeuten,

$R_8$ H, $(C_1-C_4)$Alkyl, $(C_2-C_3)$Alkenyl, $(C_2-C_3)$Alkinyl, gerade oder verzweigt, Hydroxy, Nitro, Amino, $(C_1-C_4)$Alkoxy, Mercapto, $(C_1-C_4)$Alkylthio, Hydroxy-$(C_1-C_4)$alkyl, Mercapto-$(C_1-C_4)$alkyl, F, Cl, Br, Amino-$(C_1-C_4)$alkyl, Sulfonamido, Methylendioxy, Fluor-$(C_1-C_4)$alkyl, Chlor-$(C_1-C_4)$alkyl, Brom-$(C_1-C_4)$alkyl, Cyano oder Trifluoromethyl;

$R_9$= H oder $CH_3$;

$R_{10}$= $(C_1-C_4)$Alkyl, gerade oder verzweigt, wobei der Alkylrest durch F, Cl, Br, $CF_3$, Phenyl oder Pyridyl substituiert sein kann;

X, Y und Z = O,S, $=NR_{11}$, $=CR_{12}$, $=CHR_{12}$

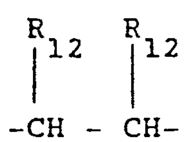 oder 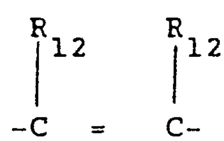

$R_{11}$= H oder $(C_1-C_4)$Alkyl, gerade oder verzweigt,

$R_{12}$= H oder zusammen mit einem vicinal stehenden Rest $R_{12}$, einen Phenylring oder für m und n = 1, dessen Dihydroform mit der Doppelbindung in Konjugation zur C-terminalen Carboxygruppe,

mit der Maßgabe,

a) daß nur einer der Reste X, Y und Z O,S

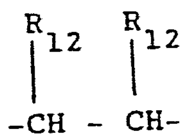

oder

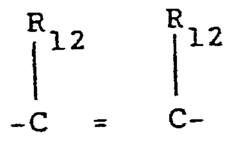

und einer oder zwei der Reste X, Y und Z $NR_{11}$ bedeuten können, und

b) wenn $R_3$ für die Gruppe $-(CH_2)_p$-NHR steht, $R_1$ Benzyl und $R_2$ H, Acetyl, Benzoyl, Pivaloyl oder einen Rest der Formel II, bedeuten

oder ihre Säureadditionssalze zur Herstellung einer pharmazeutischen Zubereitung zur Behandlung von Coronarerkrankungen oder organischem Hirnsyndrom.

2. Verwendung nach Anspruch 1, dadurch gekennzeichnet, daß in der Verbindung der Formel I

$R_1$ H, Phenyl, Benzyl, oder Phenethyl bedeutet;
$R_2$ H, Acetyl, Benzoyl, Pivaloyl, Pivaloyloxymethyl, 3-Sulfonamido-4-chlorbenzoyl, $(C_1-C_2)$alkylaminocarbonyl, $(C_1-C_2)$alkylaminothiocarbonyl oder den Rest der Formel

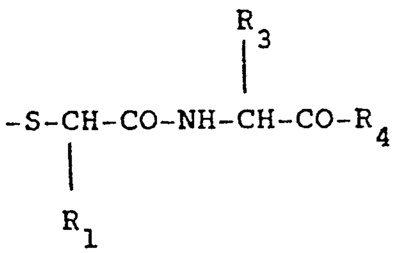

II

bedeutet;

$R_3$ wie in Anspruch 1 definiert ist.
$R_4$ den Rest einer α-Aminosäure

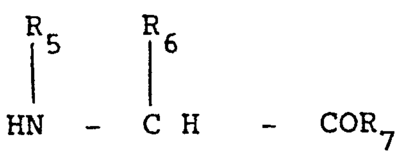

bedeutet,

worin

$R_5$ $(C_5-C_7)$-Cycloalkyl

und

$R_6$ H bedeutet

oder

$R_5$ und $R_6$ zusammen mit dem N- und dem C-Atom einen 5-, 6- oder 7-gliedrigen gesättigten Ring bilden, oder einen 4-, 5- oder 6-gliedrigen Ring, der ein oder zwei weitere der Heteroatome O,S und N enthält;

$R_7$ OH,

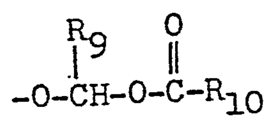

oder 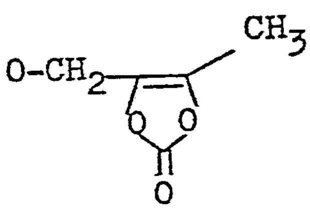

oder eine α-Aminosäure, die peptidartig mit der =CO Gruppe des Moleküls verknüpft ist,
bedeutet; worin

$R_9$ H oder $CH_3$ ist;

$R_{10}$ $(C_1-C_4)$Alkyl, gerade oder verzweigt, wobei der Alkylrest durch F, Cl, Br, $CF_3$, Phenyl oder Pyridyl substituiert sein kann.

3. Verwendung nach Anspruch 1 oder 2, dadurch gekennzeichnet, daß $R_1$ Benzyl ist.

4. Verwendung nach einem der Ansprüche 1 bis 3, dadurch gekennzeichnet, daß $R_2$ Wasserstoff, Acetyl, Benzoyl oder Pivaloyloxymethyl, vorzugsweise Wasserstoff oder Pivaloyloxymethyl ist.

5. Verwendung nach einem der Ansprüche 1 bis 4, dadurch gekennzeichnet, daß $R_4$ der Rest

$$\begin{array}{cccccc} & R_5 & & R_6 & & \\ & | & & | & & \\ - & N & - & CH & - & COR_7 \end{array}$$

ist, worin $R_5$ und $R_6$ zusammen mit dem N- und dem C-Atom einen 5-gliedrigen gesättigten Ring bilden oder einen 5-gliedrigen Ring, der als zweites Heteroatom S enthält und $R_7$ wie in Anspruch 1 oder 2 definiert ist.

6. Verwendung nach einem der Ansprüche 1 bis 5, dadurch gekennzeichnet, daß $R_7$ OH oder Pyvaloyloxymethoxy ist.

7. Verwendung nach einem der Ansprüche 1 bis 6, dadurch gekennzeichnet, daß $R_3$ $-CH_3$ ist.

8. Verwendung nach einem der Ansprüche 1 bis 6, dadurch gekennzeichnet, daß $R_3$ $-(CH_2)_4$ NHR ist, worin R wie in Anspruch 1 definiert ist, vorzugsweise H, Acetyl oder tert. Butoxycarbonyl bedeutet.

9. Verwendung nach einem der Ansprüche 1 bis 8, dadurch gekennzeichnet, daß in der Verbindung der Formel I alle asymmetrischen C-Atome in der S-Konfiguration vorliegen.

10. Verwendung nach Anspruch 1, dadurch gekennzeichnet, daß die Verbindung der Formel I

N-[N-(3-Phenyl-2-S-pivaloyloxymethylmercaptopropanoyl)-S-alanyl]-S-prolin-pivaloyloxymethylester;

N-[N-(3-Phenyl-2-S-benzoylmercaptopropanoyl)-S-alanyl]-S-prolin;

N-[N-(3-Phenyl-2-S-acetylmercaptopropanoyl)-S-alanyl]-S-prolin-pivaloyloxymethylester; oder

N-[N-(3-Phenyl-2-S-mercaptopropanoyl)-S-alanyl]-S-prolin-pivaloyloxymethylester ist.

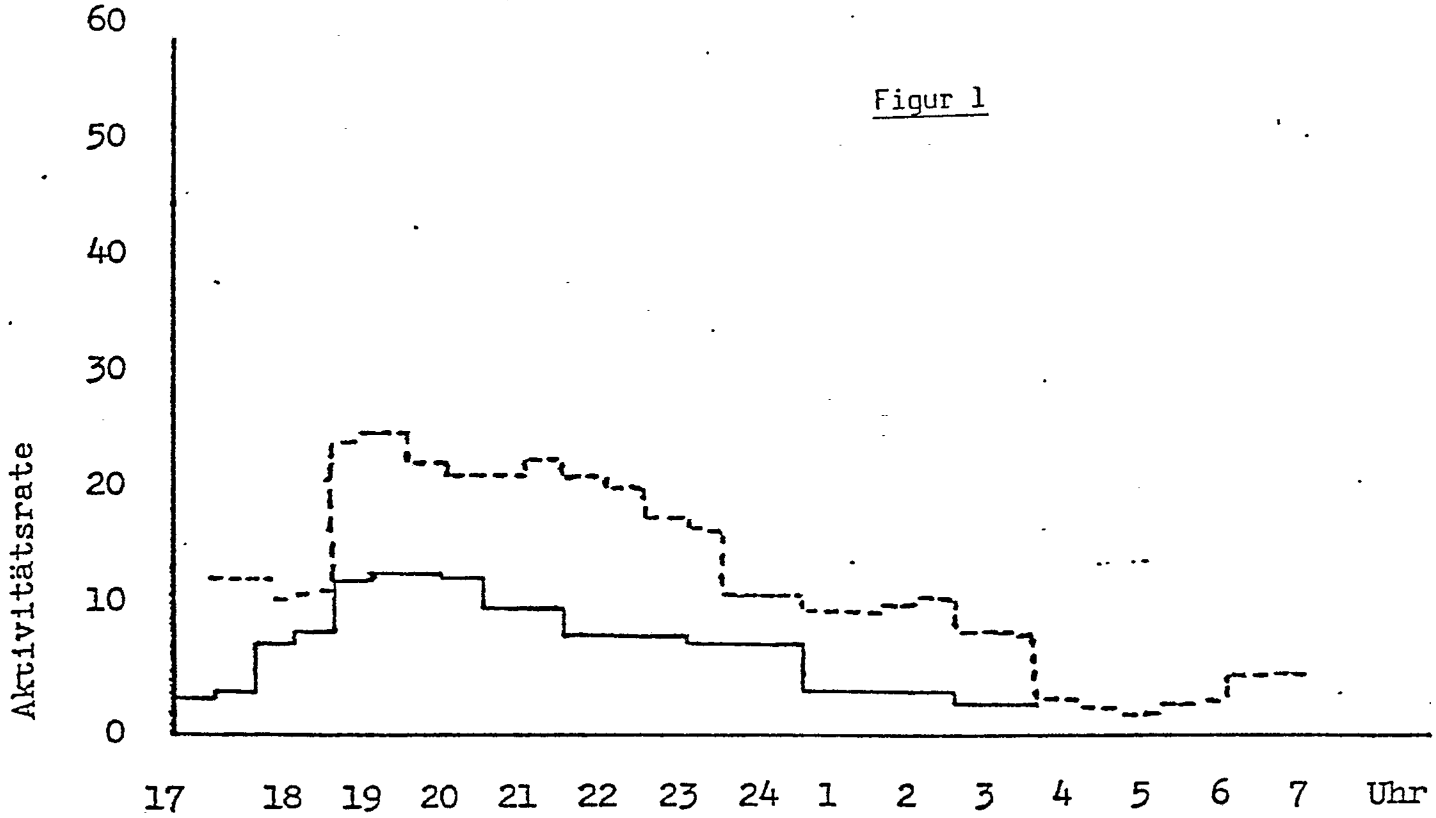


——— Tylose (Kontrolle) 10 mg/kg

----- N-[N-(3-Phenyl-2-S-acetyl-mercaptopropanoyl)-S-anamyl]-S-prolin-pivaloyloxymethylester 10 mg/kg